# EUROPÄISCHE PATENTSCHRIFT

(11) **EP 3 325 012 B1**
(45) Veröffentlichungstag und Bekanntmachung des Hinweises auf die Patenterteilung: **16.10.2019**
(21) Anmeldenummer: 16732559.6
(22) Anmeldetag: 20.06.2016
(51) Int. Cl.: A61K 45/06, A61K 31/352, A61K 31/573, A61P 11/02

(54) **CINEOLHALTIGE ZUSAMMENSETZUNG ZUR BEHANDLUNG VON NASENPOLYPEN**
CINEOLE-CONTAINING COMPOSITION FOR TREATING NOSE POLYPS
COMPOSITION CONTENANT DU CINÉOL POUR LE TRAITEMENT DE POLYPES NASAUX

(30) Priorität: 23.10.2015 DE 102015013667; 26.10.2015 DE 102015013921; 12.11.2015 DE 102015119541
(43) Veröffentlichungstag der Anmeldung: 30.05.2018
(73) Patentinhaber: Maria Clementine Martin Klosterfrau Vertriebsgesellschaft mbH, 50670 Köln (DE)
(72) Erfinder: UNKAUF, Markus, 80538 München (DE); WOLLENBERG, Barbara, 23566 Lübeck (DE); PRIES, Ralph, 23619 Rehhorst (DE)
(74) Vertreter: Von Rohr Patentanwälte Partnerschaft mbB
(86) Internationale Anmeldenummer: PCT/EP2016/064161
(87) Internationale Veröffentlichungsnummer: WO 2017/067677

(56) Entgegenhaltungen:
- TRIPTI MISHRA ET AL: "Composition and in vitro cytotoxic activities of essential oil of Hedychium spicatum from different geographical regions of western Himalaya by principal components analysis", NATURAL PRODUCT RESEARCH, Bd. 30, Nr. 10, 21. Juli 2015 (2015-07-21) , Seiten 1224-1227, XP055296775, GB ISSN: 1478-6419, DOI: 10.1080/14786419.2015.1049176 & Tripti Mishra ET AL: "SUPPLEMENTARY MATERIAL Composition and in vitro cytotoxic activities of essential oil of Hedychium spicatum from different geographical region of Western Himalaya by principal component analysis", , 21. Juli 2015 (2015-07-21), Seiten 1-10, XP055296780, Gefunden im Internet: URL:http://www.tandfonline.com/doi/suppl/1 0.1080/14786419.2015.1049176/suppl_file/gn pl_a_1049176_sm3874.pdf [gefunden am 2016-08-22]
- PÄR STJÄRNE ET AL: "A Randomized Controlled Trial of Mometasone Furoate Nasal Spray for the Treatment of Nasal Polyposis", ARCHIVES OF OTOLARYNGOLOGY HEAD AND NECK SURGERY., Bd. 132, Nr. 2, Februar 2006 (2006-02), Seite 179, XP055297649, US ISSN: 0886-4470, DOI: 10.1001/archotol.132.2.179

## Beschreibung

Die vorliegende Erfindung betrifft das technische Gebiet der Behandlung bzw. Therapie von Nasenpolypen (medizinisch und synonym auch als *Polyposis nasi et sinuum* oder verkürzt nur als *Polyposis nasi* bezeichnet).

Insbesondere betrifft die vorliegende Erfindung eine Zusammensetzung, insbesondere eine pharmazeutische Zusammensetzung, bzw. ein Arzneimittel zur prophylaktischen und/oder therapeutischen Behandlung von Nasenpolypen (*Polyposis nasi et sinuum*) und die entsprechende Verwendung dieser Zusammensetzung bzw. dieses Arzneimittels für den genannten therapeutischen Zweck.

Weiterhin betrifft die vorliegende Erfindung eine Wirkstoffkombination, insbesondere in Form eines Kit (Kit-of-Parts), zur prophylaktischen und/oder therapeutischen Behandlung von Nasenpolypen (*Polyposis nasi et sinuum*).

Schließlich betrifft die vorliegende Erfindung die Verwendung von Cineol, insbesondere 1,8-Cineol, zur Regulation des Wnt-Signalwegs, insbesondere Wnt/beta-Catenin-Signaltransduktionswegs, zur prophylaktischen und/oder therapeutischen Behandlung von Nasenpolypen (*Polyposis nasi et sinuum*), bzw. die Verwendung von Cineol, insbesondere 1,8-Cineol, zur prophylaktischen und/oder therapeutischen Behandlung von mit dem Wnt-Signalweg, insbesondere Wnt/beta-Catenin-Signaltransduktionsweg, in Zusammenhang stehenden und/oder hierdurch induzierten und/oder begünstigten Erkrankungen, insbesondere von Nasenpolypen (*Polyposis nasi et sinuum*).

Nasenpolypen sind insbesondere weiche, im Allgemeinen gutartige (benigne) Wucherungen der Nasenschleimhaut, welche in der Regel bis in die Nasenhöhlen hineinragen. Sie sprießen sozusagen aus der Schleimhaut der Nase, mit welcher sie über eine Art Stiel verbunden sind. Manche Nasenpolypen sind nur wenige Millimeter groß und stören nicht bzw. kaum, wohingegen andere zu ausgedehnten Gebilden heranwachsen können, welche dann sogar die Nasenräume verlegen können. Da dies die Nasenatmung einschränkt, kann die Erkrankung sehr lästig werden und die Lebensqualität enorm einschränken. Nasenpolypen können - abgesehen von einer Behinderung der Nasenatmung - zudem auch zu einem Geruchssinnverlust und weiteren Erkrankungen der Nasennebenhöhen führen, wie nachfolgend noch ausgeführt.

Patienten mit Nasenpolypen sind also meist durch eine eingeschränkte Nasenatmung beeinträchtigt: Diese Patienten bekommen durch die Nase nicht ausreichend Luft und atmen dadurch öfter durch den Mund. Die Betroffenen schnarchen deswegen häufig und leiden oftmals unter Schlafstörungen, so dass insgesamt ihre Leistungsfähigkeit eingeschränkt ist. Außerdem wird die durch den Mund geatmete Luft nicht gefiltert, so dass die allgemeine Infektionsgefahr erhöht ist. Betroffene haben oftmals das Gefühl, ständig eine sogenannte verstopfte Nase zu haben, was auch zu einer näselnden Stimme führen kann. Außerdem leiden diese Patienten häufig an wiederkehrenden Entzündungen der Nasennebenhöhlen (*Sinusitis*) und dumpfen Kopfschmerzen. Darüber hinaus kann ihr Riechvermögen eingeschränkt sein und dadurch auch der Geschmackssinn, was wiederum einen Verlust an Lebensqualität nach sich zieht. Nasenpolypen bei Kindern können zudem auch eine Mittelohrentzündung (*Otitis media*) verursachen.

Nasenpolypen sind aus pathophysiologischer Sicht also im Allgemeinen blass-grau gestielte Ausstülpungen von chronisch entzündeter und ödematöser Schleimhaut in der Nasenhöhle, welche überwiegend aus dem Siebbeinbereich, mittleren Nasengang und der mittleren Nasenmuschel imponieren d. h. also benigne Wucherungen, welche von einer anhaltend entzündeten Nasenschleimhaut ausgehen. Diese Ausstülpungen bestehen im Allgemeinen aus Schleimhaut, welche insbesondere aus den Siebbeinzellen oder aus den Kieferhöhlen stammt. Mikroskopisch sind Flüssigkeitseinlagerungen und körpereigene Entzündungszellen zu beobachten. Als Ursprungslokalisation konnte die Schleimhaut der mittleren Nasenmuschel sowie des mittleren Nasengangs identifiziert werden, wobei noch ungeklärt ist, warum die untere Nasenmuschel im Allgemeinen nicht zur Polypenbildung neigt.

Nasenpolypen sind histologisch durch Ödem und/oder Fibrose, eine verminderte Vakularisation sowie eine verminderte Anzahl der Drüsen und Nervenendigungen bei oftmals geschädigtem Epithel gekennzeichnet. Bei der histologischen Aufarbeitung von Nasenpolypen lässt sich eine grobe Trennung in eosinophile Polypen, welche etwa 65 bis 90 % der Fälle des Krankenguts entsprechen, und neutrophile Polypen beschreiben. Der vermehrten Gewebseosinophilie liegt eine gesteigerte transendotheliale Migration und eine Inhibierung des programmierten Zelltodes in Eosinophilen zugrunde.

Klinisch geht die durch eine von Eosinophilen dominierte Entzündung gekennzeichnete *Polyposis nasi et sinuum* in bis zu 25 % der Fälle mit einer Acetylsalicylsäure-Intoleranz einher. In bis zu 40 % ist die *Polyposis nasi et sinuum* mit einem Asthma bronchiale assoziiert. Treten diese Faktoren gemeinsam auf, spricht man von einer Samter- oder Widal-Trias. Gesicherte Zusammenhänge werden auch zwischen der eosinophilen *Polyposis nasi et sinuum* und dem Churg-Strauss-Syndrom, einer eosinophilen Immunvaskulitis, beschrieben.

Der genaue Entstehungsmechanismus von Nasenpolypen ist noch unbekannt: Ein Nasenpolyp wächst meist aus einer der Nasennebenhöhlen heraus in die Nasenhaupthöhle. Hauptsächlich entstehen die Nasenpolypen in der Kieferhöhle (*Sinus maxillaris*) oder den Siebbeinzellen (*Cellulae ethmoidales*). Sie treten aus den Ausführungsgängen aus und liegen meistens im mittleren Nasengang unter der mittleren Nasenmuschel.

Nasenpolypen sind zwar weit verbreitet. Die genauen Ursachen ihrer Entstehung ist jedoch noch ungeklärt. Bislang ist bekannt, dass einige Basiserkrankungen existieren, bei welchen Betroffene häufiger Nasenpolypen entwickeln (z. B. Patienten mit *Asthma bronchiale*, von denen bis zu 40 Prozent auch unter Nasenpolypen leiden, oder aber Patienten mit allergischer Reaktion in Bezug auf Acetylsalicylsäure, von denen etwa 25 % Nasenpolypen entwickeln).

Ein weiterer Risikofaktor für die Bildung von Nasenpolypen scheint auch eine allgemeine Neigung (Disposition) der Schleimhaut zu Entzündungen zu sein. Als andere Risikofaktoren für die Entstehung von Nasenpolypen gelten zudem die zuvor erwähnte Allergie gegen Schmerzmittel, *Asthma bronchiale*, Bronchitis, Kartagener-Syndrom und Mukoviszidose (zystische Fibrose).

Für weitergehende Einzelheiten zu dem Krankheitsbild der Nasenpolypen (*Polyposis nasi et sinuum*) kann beispielsweise auch verwiesen werden auf Pschyrembel, Medizinisches Wörterbuch, 257. Auflage, 1993, Verlag Walter de Gruyter, Stichworte "Nasenpolypen" und "Polyposis nasi", sowie auf Roche Lexikon Medizin, 3. Auflage, 1993, Urban & Schwarzenberg Verlag, Stichworte "Nasenpolyp" und "Polyposis".

Eine Behandlung von Nasenpolypen (*Polyposis nasi et sinuum*), insbesondere eine nachhaltige Behandlung, erweist sich auf Basis herkömmlicher Therapiemethoden oftmals als diffizil und wenig effizient, insbesondere aufgrund der hohen Rezidivraten, und ist für die betroffenen Patienten meist mit unerwünschten Nebenwirkungen und Unannehmlichkeiten verbunden.

Je nachdem, wie stark die Beschwerden durch die Nasenpolypen sind, wird entweder eine konservative, insbesondere medikamentöse Therapie oder eine operative Behandlung der Nasenpolypen durchgeführt.

Da Nasenpolypen langsam wachsen, gewöhnen sich Patienten oft an die Symptome und suchen in der Regel erst zu einem relativ späten Zeitpunkt ärztliche Hilfe auf. Mit den herkömmlichen Therapieformen können zwar die akuten Beschwerden gelindert werden.

So kann beispielweise eine operative Entfernung der Nasenpolypen häufig schnell die akuten Beschwerden bessern; dennoch bilden sich bei etwa 75 % der auf diese Weise behandelten Patienten in den ersten Jahren nach der Entfernung erneut Nasenpolypen. Deshalb ist es im Stand der Technik vorgesehen, auch nach einer Nasenpolypenoperation langandauernd Kortikosteroide topisch (z. B. als kortisonhaltige Nasensprays) oder aber auch systemisch anzuwenden (einhergehend mit einer Vielzahl unerwünschter Nebenwirkungen), um zu verhindern, dass erneut Polypen in der Nase auftreten (was aber oftmals nicht erreicht wird).

Gering ausgeprägte Erkrankungen können dagegen zunächst konservativ durch topische und/oder systemische Verabreichung von Kortikosteroiden bzw. Glukokortikoiden behandelt werden. Kortikosteroide bzw. Glukokortikoide wirken entzündungshemmend und lindern auf diese Weise die Entzündungsreaktionen, zu denen auch das Anschwellen der Nasenschleimhaut zählt. Bei einer solchen Kortikosteroidtherapie sind die Kortikosteroide regelmäßig über mehrere Monate hinweg anzuwenden. Häufig bessern sich die Symptome erst nach einigen Wochen der Anwendung.

Insbesondere die mit einer Eosinophilie einhergehenden Nasenpolypen zeichnen sich durch eine schwere Entzündungsreaktion aus, welche durch Zytokine (wie IL-5) und Chemokine (wie Eotaxin und RANTES) gekennzeichnet ist. Kortikosteroide wirken typischerweise besonders eindrucksvoll bei eosinophileassoziierten Entzündungen, weswegen der Parameter "Eosinophilie" häufig als Indikator für den Einsatz dieser Arzneimittel bezeichnet wird. So konnte belegt werden, dass Steroide die Apoptose dieser Entzündungszellen induzieren, wobei die Stärke des supprimierenden Effektes auf die IL-5 Produktion aus T-Zellen ein entscheidender Parameter sein dürfte. Die symptomatische Therapie bei Nasenpolypen konnte bereits durch Studien mit den ersten topisch zu verabreichenden Kortikosteroiden etabliert werden. In den letzten Jahren wurden die klinischen Beobachtungen zunehmend durch objektive Parameter gestützt (Rhinomanometrie, Rhinometrie, Peak Nasal Inspiratory Flow PNIF, Magnetresonanz-Tomographie). Auch die Rezidivraten bzw. der Zeitpunkt der Rezidive nach Operation konnten in einigen Studien verbessert bzw. herausgezögert werden, wobei jedoch die Untersuchungen allerdings über den Zeitraum eines Jahres nicht hinausgehen. Die in den Studien eingesetzte Dosierung der topischen Steroide liegt häufig über der für die allergische Rhinitis empfohlenen Dosierung.

Zumeist ist jedoch eine chirurgische Therapie (z. B. eine Infundibulotomie) erforderlich, um die Nasenatmungsbehinderung und den Geruchsverlust wirksam zu behandeln. Derartige Operationen werden insbesondere endonasal und bevorzugt minimal-invasiv durchgeführt und können Heilungsraten von bis zu etwa 50 % und eine Beschwerdebesserung in bis zu etwa 90 % der Fälle bringen. Neben der Entfernung der Nasenpolypen werden üblicherweise zusätzlich auch die Ausführungsgänge der Nasennebenhöhlen erweitert, so dass diese besser belüftet werden und sich weniger leicht entzünden sollen. Da aber Nasenpolypen und chronisch-polypöse Nebenhöhlenerkrankungen zu Rezidiven neigen, sind im Krankheitsverlauf oftmals mehrere Operationen erforderlich. Derartige minimal-invasive Nebenhöhlenoperationen zählen zu den anspruchsvollen Eingriffen im HNO-Bereich und sind für die betroffenen Patienten vielfach mit einem mehrtägigen Krankenhausaufenthalt und mit intensiver Nachbehandlung verbunden, welche insbesondere den Einsatz von topischen Kortikosteroiden über mehrere Monate als Behandlungsversuch zur Rezidivprophylaxe umfasst. Unter einer mehrmonatigen postoperativen Steroidtherapie scheint es auch zu einer verbesserten Wundheilung nach einer chirurgischen Therapie zu kommen; kontrollierte Untersuchungen zur Wundheilung nach Sinusoperationen liegen jedoch nicht vor.

Für weitergehende Einzelheiten zur Therapie von Nasenpolypen (*Polyposis nasi et sinuum*) kann beispielsweise auch verwiesen werden auf die relevanten Leitlinien "Rhinosinusitis" und "Diagnose und Therapie der Sinusitis und Polyposis nasi" der Deutschen Gesellschaft für Hals-Nasen-Ohren-Heilkunde, Kopf- und Hals-Chirurgie.

Die Therapiekonzepte des Standes der Technik greifen also entweder auf operative Methoden zur Entfernung der Nasenpolypen und/oder kortikosteroidbasierte konservative Therapiekonzepte zurück, wobei diese Methoden des Standes der Technik mit erheblichen Unannehmlichkeiten und Nebenwirkungen für die betroffenen Patienten verbunden sind. Insbesondere kann mit den Therapiekonzepten des Standes der Technik oftmals eine Rezidivierung der Nasenpolypen, insbesondere auch nach postoperativen Zuständen, nicht in effizienter Weise verhindert werden, so dass betroffene Patienten sich mehrmals operativen Eingriffen rezidivierender Nasenpolypen unterziehen müssen. Zudem sind konservative Therapiekonzepte auf Basis von Kortikosteroiden, insbesondere bei postoperativen Zuständen, gerade bei der Behandlung von Kindern, insbesondere Kleinkindern, wegen des nicht unerheblichen Nebenwirkungsprofils dieser Wirkstoffe unerwünscht.

Im Zusammenhang mit Therapiekonzepten des Standes der Technik beschreibt die FR 2 956 817 A für eine therapeutische Verwendung in der Prävention oder der Behandlung von Keratosen im Transformationsstadium, präkanzerösen (prämaligne) oder kanzerösen Keratinozyten oder von aus der Transformation einer Keratose stammenden Karzinomen eine Zusammensetzung, welche mindestens einen Bestandteil von essentiellen Ölen der Pflanzen *Oregano compactum* oder *Aniba rosaeodora* umfasst.

Weiterhin betrifft die wissenschaftliche Publikation gemäß T. Nishra et al., "Composition and in vitro cytotoxic activities of essential oil of Hedychium spicatum from different geographical regions of western Himalaya by principal components analysis", Natural Product Research, Band 30, Nr. 10, 21. Juli 2015, Seiten 1224 bis 1227 nur Zusammensetzungen und *In-vitro*-Untersuchungen zur zytotoxischen Aktivität ätherischer Öle von *Hedychium spicatum* aus verschiedenen geographischen Regionen des westlichen Himalaya-Gebirges mittels Analyse der Hauptkomponenten.

Das der vorliegenden Erfindung zugrundeliegende Problem besteht daher in der Bereitstellung eines neuartigen Therapiekonzepts für die prophylaktische und/oder therapeutische Behandlung von Nasenpolypen (*Polyposis nasi et sinuum*), wobei die zuvor geschilderten Nachteile des Standes der Technik zumindest weitgehend vermieden oder aber wenigstens abgeschwächt werden sollen.

Insbesondere soll eine solches Therapiekonzept gegenüber herkömmlichen Therapiemethoden mit weniger Nebenwirkungen und/oder Unannehmlichkeiten für die betroffenen Patienten verbunden sein und/oder eine mindestens vergleichbare, bevorzugt sogar verbesserte Wirkeffizienz bereitstellen.

Zur Lösung des zuvor geschilderten Problems schlägt die vorliegende Erfindung - gemäß einem **ersten** Aspekt der vorliegenden Erfindung - eine cineolhaltige Zusammensetzung, insbesondere eine cineolhaltige pharmazeutische Zusammensetzung, nach Anspruch 1 vor; weitere, insbesondere vorteilhafte Ausgestaltungen der erfindungsgemäßen Zusammensetzung sind Gegenstand der diesbezüglichen Unteransprüche.

Gleichermaßen betrifft die vorliegende Erfindung - gemäß einem **zweiten** Aspekt der vorliegenden Erfindung - ein Arzneimittel zur prophylaktischen und/oder therapeutischen Behandlung von Nasenpolypen (*Polyposis nasi et sinuum*) bzw. zur Verwendung bei der prophylaktischen und/oder therapeutischen Behandlung von Nasenpolypen (*Polyposis nasi et sinuum*) gemäß dem diesbezüglichen Anspruch (Anspruch 13).

Des Weiteren betrifft die vorliegende Erfindung - gemäß einem weiteren, **dritten** Aspekt der vorliegenden Erfindung - eine Wirkstoffkombination, insbesondere in Form eines Kit (Kit-of-parts), zur prophylaktischen und/oder therapeutischen Behandlung von Nasenpolypen (*Polyposis nasi et sinuum*) bzw. zur Verwendung bei der prophylaktischen und/oder therapeutischen Behandlung von Nasenpolypen (*Polyposis nasi et sinuum*) gemäß dem diesbezüglichen Anspruch (Anspruch 14).

Schließlich betrifft die vorliegende Erfindung - gemäß einem nochmals weiteren, **vierten** Aspekt der vorliegenden Erfindung - Cineol, insbesondere 1,8-Cineol, zur Verwendung bei der prophylaktischen und/oder therapeutischen Behandlung von mit dem Wnt-Signalweg, insbesondere Wnt/beta-Catenin-Signaltransduktionsweg, in Zusammenhang stehenden und/oder hierdurch induzierten und/oder begünstigten Erkrankungen, insbesondere von Nasenpolypen (*Polyposis nasi et sinuum*), vorzugsweise von rezidivierenden Formen von Nasenpolypen (*Polyposis nasi et sinuum*), gemäß dem diesbezüglichen Anspruch (Anspruch 15).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine Zusammensetzung, insbesondere pharmazeutische Zusammensetzung, zur Verwendung bei der prophylaktischen und/oder therapeutischen Behandlung von Nasenpolypen (*Polyposis nasi et sinuum*), wobei die Zusammensetzung als Wirkstoff, insbesondere pharmazeutischen Wirkstoff, Cineol, vorzugsweise 1,8-Cineol, enthält.

Die vorliegende Erfindung bzw. das erfindungsgemäße Therapiekonzept zur Behandlung von Nasenpolypen ist mit einer Vielzahl von Vorteilen und Besonderheiten verbunden, welche nachfolgend - in nicht beschränkender, kein Anspruch auf Vollständigkeit erhebender Weise - ausgeführt sind:
Im Rahmen der vorliegenden Erfindung kann erstmals ein neuartiges Therapiekonzept für die prophylaktische und/oder therapeutische Behandlung von Nasenpolypen (*Polyposis nasi et sinuum*) bereitgestellt werden, mit welchem die zuvor geschilderten Nachteile des Standes der Technik zumindest weitgehend vermieden oder aber wenigstens abgeschwächt werden können. Insbesondere ist das erfindungsgemäße Therapiekonzept gegenüber herkömmlichen Therapiemethoden mit weniger Nebenwirkungen und/oder Unannehmlichkeiten für die betroffenen Patienten verbunden, wobei im Rahmen der vorliegenden Erfindung eine mindestens vergleichbare, bevorzugt sogar eine verbesserte Wirkeffizienz im Vergleich zu den Therapiekonzepten des Standes der Technik bereitgestellt wird.

Wie die Anmelderin überraschenderweise herausgefunden hat, lassen sich mit dem Wirkstoff Cineol, insbesondere 1,8-Cineol, bei der Behandlung von Nasenpolypen nahezu vergleichbare Ergebnisse wie bei der Verwendung von Glukokortikoiden erzielen, wobei Cineol als natürlicher Wirkstoff aber nicht die mit Kortikosteroiden verbundenen schwerwiegenden Nebenwirkungen aufweist. Dabei kann das Cineol als Monotherapeutikum oder aber auch bei der Behandlung schwerwiegenderer Fälle auch in Kombination mit Kortikosteroiden eingesetzt werden, wobei durch die gemeinsame bzw. kombinierte Verwendung von Cineol deren Wirkung sogar verstärkt wird bzw. deren Dosis nicht unerheblich reduziert werden kann (und dies bei gleicher Wirkeffizienz).

Wie nachfolgend dargelegt, kann insbesondere bei der Behandlung von postoperativen Zuständen die Rezidivrate signifikant verringert werden.

Dabei ist auch zu berücksichtigen, dass der Wirkstoff Cineol im Rahmen der vorliegenden Erfindung topisch (z. B. intranasal und/oder systemisch (z. B. peroral)) verabreicht werden kann. Sowohl bei topischer Anwendung, bei welcher das Cineol im Allgemeinen unmittelbar auf die Nasenschleimhäute aufgebracht wird, als auch bei systemischer (z. B. peroraler) Anwendung kommt dabei das antiphlogistische, kortikosteroidartige Wirkprofil von Cineol zum Tragen.

Wie die Anmelderin weiterhin überraschend herausgefunden hat, lassen sich mit dem erfindungsgemäßen Konzept insbesondere rezidivierende Formen von Nasenpolypen in effizienter Weise behandeln, welche bei der Anwendung herkömmlicher Therapiekonzepte bislang nicht bzw. nicht immer zufriedenstellend behandelt werden konnten.

Wie nachfolgend gleichermaßen noch ausgeführt wird, lassen sich mit dem erfindungsgemäßen Therapiekonzept gerade auch im Zusammenhang mit dem Wnt-Signal, insbesondere Wnt/beta-Catenin-Signaltransduktionsweg, im Zusammenhang stehende Formen, insbesondere rezidivierende Formen, von Nasenpolypen behandeln, welche durch herkömmliche Therapiekonzepte des Standes der Technik bislang nicht in wirksamer Weise zufriedenstellend therapiert werden konnte.

Insgesamt stellt daher die vorliegende Erfindung ein effizientes und flexibel einsetzbares Therapiekonzept bereit, welches auf einen natürlichen Wirkstoff (nämlich Cineol, insbesondere 1,8-Cineol) zurückgreift, mit welchem keine nennenswerten unerwünschten Nebenwirkungen verbunden sind. Insbesondere kann das erfindungsgemäße Therapiekonzept in effizienter und flexibler Weise auch mit herkömmlichen Therapiekonzepten kombiniert werden; so kann beispielsweise das erfindungsgemäße Therapiekonzept nach einer chirurgischen Entfernung von Nasenpolypen zur Verhinderung der Rezidivbildung eingesetzt werden, oder aber es kann das erfindungsgemäße Therapiekonzept mit herkömmlichen, systemisch und/oder topisch applizierten Kortikosteroiden kombiniert werden, insbesondere mit der Maßgabe, deren Wirkweise zu verstärken bzw. deren Dosis zu reduzieren (und damit auch das Nebenwirkungsprofil dieser Kortikosteroide).

Was den im Rahmen der vorliegenden Erfindung zum Einsatz kommenden natürlichen Wirkstoff Cineol, insbesondere 1,8-Cineol anbelangt, so ist diesbezüglich Folgendes auszuführen:
1,8-Cineol gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und einem Siedepunkt von 176 bis 177 °C, welche in Wasser unlöslich, aber mit den meisten organischen Lösemitteln mischbar ist. Natürlich kommt 1,8-Cineol als Hauptbestandteil des Eukalyptusöls (Eukalyptusöl enthält bis zu 85 Gew.-% 1,8-Cineol), aber auch in anderen Pflanzen vor, so z.B. in Minze, Heilsalbei, Thymian, Basilikum und im Teebaum. Da-rüber hinaus ist 1,8-Cineol beispielsweise in Niaouli-, Juniperus-, Piper-, Cannabis-, Kajeput-, Salbeiöl, Myrtenöl und anderen ätherischen Ölen enthalten. Technisches 1,8-Cineol, welches im Allgemeinen 99,6- bis 99,8-%ig ist, wird im Allgemeinen durch fraktionierte Destillation von Eukalyptusöl gewonnen. 1,8-Cineol wird im Stand der Technik insbesondere als Expektorans bei Bronchialkatarrhen und anderen Atemwegserkrankungen vorwiegend in der Veterinärmedizin, aber darüber hinaus auch als Aromastoff in der Parfümindustrie angewendet. Darüber hinaus wird 1,8-Cineol in der Zahnmedizin bei der Revision von Wurzelfüllungen verwendet.

In pharmakologischer Hinsicht wirkt 1,8-Cineol in den oberen und unteren Atemwegen, insbesondere in der Lunge und den Nasennebenhöhlen, schleimlösend und bakterizid. Außerdem hemmt es bestimmte Neurotransmitter, welche z. B. für die Verengung von Bronchien verantwortlich sind (Bei chronischobstruktiven Lungenerkrankungen und Asthma bronchiale kann daher durch Gabe von reinem 1,8-Cineol die Lungenfunktion verbessert werden.). Aufgrund seiner kortikosteroidartigen Wirkung kann Cineol als Substitut zu oder in Co-Medikation mit Kortikosteroiden angewendet werden. 1,8-Cineol kann - wie nachfolgend noch ausgeführt - grundsätzlich sowohl topisch, z. B. (intra)nasal und/oder inhalativ, oder aber systemisch, insbesondere peroral (z. B. in Form von Kapseln), angewendet werden.

Als Wirkstoff besitzt 1,8-Cineol also schleimlösende wie entzündungshemmende Wirkungen. Bei systemischer Anwendung wird 1,8-Cineol leicht resorbiert und gelangt über die Blutbahn in den Atmungsorganen zur Wirkung. 1,8-Cineol kann auf diese Weise beispielsweise entzündliche Sekrete sowie zähen Schleim in den Luftwegen verflüssigen und entzündlichen Prozessen in den Atemwegen entgegenwirken. Im Bereich der oberen Luftwege schwinden die Behinderung der Nasenatmung bei Schnupfen und die Benommenheit des Kopfes.

Für weitergehende Einzelheiten zu dem Wirkstoff 1,8-Cineol kann beispielsweise verwiesen werden auf Römpp Chemielexikon, Georg Thieme Verlag, Stuttgart/New York, 10. Auflage, Band 1, 1996, Seite 752, Stichwort: "Cineol", sowie die dort referierte Literatur.

Überraschenderweise hat nunmehr die Anmelderin im Rahmen ihrer langjährigen wissenschaftlichen Studien eine weitere medizinische Indikation aufgefunden, welche mit dem naturbasierten Wirkstoff Cineol, insbesondere 1,8-Cineol, in effizienter und nebenwirkungsfreier Weise therapiert werden kann, nämlich Nasenpolypen (*Polyposis nasi et sinuum*), insbesondere auch zu Rezidivbildung neigende Formen von Nasenpolypen.

Insbesondere kann im Rahmen der vorliegenden Erfindung die erfindungsgemäße Zusammensetzung und/oder das Cineol zur prophylaktischen und/oder therapeutischen Behandlung von rezidivierenden Formen von Nasenpolypen (*Polyposis nasi et sinuum*) eingesetzt werden. Derartige Erkrankungsformen sind mit herkömmlichen Therapiemethoden nicht zufriedenstellend therapierbar.

Gemäß einer besonderen Ausführungsform der vorliegenden Erfindung wird die erfindungsgemäße Zusammensetzung und/oder das Cineol zur prophylaktischen und/oder therapeutischen Behandlung von mit dem Wnt-Signalweg, insbesondere Wnt/beta-Catenin-Signaltransduktionsweg, in Zusammenhang stehenden Formen von Nasenpolypen (*Polyposis nasi et sinuum*), vorzugsweise zur prophylaktischen und/oder therapeutischen Behandlung von über den Wnt-Signalweg, insbesondere Wnt/beta-Catenin-Signaltransduktionsweg, induzierten und/oder begünstigten Formen von Nasenpolypen (*Polyposis nasi et sinuum*), eingesetzt.

Derartige Formen bzw. Zustände von Nasenpolypen können bislang mit herkömmlichen Therapiekonzepten nicht bzw. nicht immer zufriedenstellend behandelt oder therapiert werden.

Der sogenannte Wnt-Signalweg, insbesondere Wnt/beta-Catenin-Signaltransduktionsweg, ist einer von vielen Signaltransduktionswegen, mittels welchem Zellen auf äußere Signale reagieren können. Dieser Signalweg ist nach seinem Liganden "Wnt" benannt, einem Signalprotein, welches als lokaler Mediator eine wichtige Funktion bei der Entwicklung verschiedener tierischer Zellen einnimmt.

Das Akronym "Wnt" selbst ist historisch begründet und setzt sich aus den Bestandteilen "Wg" einerseits und "Int-1" andererseits zusammen: Der Akronymteil "W" entstammt dem Kürzel "Wg", welches wiederum für das englische Wort "wingless" (flügellos) steht und denjenigen Wachstumsfaktor der Wnt-Familie bezeichnet, welcher als erster entdeckt wurde (Wenn das Wg-Gen mutiert ist, entsteht bei der Taufliege *Drosophila melanogaster* eine flügellose Variante.). Das Akronymteil "nt" stammt von dem Int-Gen, welches bei der Maus die Entwicklung von Brustkrebs fördert bzw. triggert, wenn es aktiviert ist. Insgesamt wurden inzwischen 19 verschiedene Wnt-Gene identifiziert.

An der Signaltransduktion des Wnt-Signalweges sind zahlreiche Proteine beteiligt. Er ist essentiell für die normale Embryonalentwicklung (Embryogenese) und wird auch bei bestimmten Krebsformen beobachtet. Nach derzeitigem Wissensstand bindet das Wnt-Signalprotein an den Rezeptor "*Frizzled*" (zusammen mit dem Co-Rezeptor "LRP" [*low-density lipoprotein receptor-related protein*]), welcher das Protein "*Dishevelled* (DVL)" aktiviert, welches wiederum inhibierend auf einen Proteinkomplex (bestehend aus GSK-3 [einer Proteinkinase], dem Tumorsupressorprotein APC und dem Protein Axin-1) einwirkt, welcher normalerweise beta-Catenin abbaut. Da nun die Degradation von beta-Catenin inhibiert ist, sammelt sich dieses im Cytoplasma und im Zellkern an. Im Zellkern bildet das beta-Catenin mit TCF/LEF einen Protein-Komplex und aktiviert auf diese Weise spezifische Zielgene. Das APC-Gen ist ein Tumorsuppressorgen, welches zuerst bei einem bestimmten Typ von Darmkrebs mutiert gefunden wurde, jedoch kann dieses Gen auch bei anderen Krebsarten mutieren; das APC-Gen kodiert für das APC-Protein, welches im Wnt-Signalweg an der Degradation von beta-Catenin beteiligt ist.

Der Wnt-Signalweg ist bei vielen Vorgängen im Rahmen der Embryonalentwicklung (Embryogenese) von Bedeutung, wie z. B. bei der Ausbildung der Körperachse und der Bildung von Organanlagen. In adulten Zellen hingegen ist er zumeist inaktiv, in Tumorzellen kann er aber wieder aktiviert vorliegen.

In Zellen, in welchen der Wnt-Signalweg inaktiv ist, liegt beta-Catenin in einem Komplex gebunden vor, welcher dazu führt, dass beta-Catenin ständig abgebaut wird; auf diese Weise wird beta-Catenin daran gehindert, die Transkription bestimmter Gene zu aktivieren. Dieser Komplex, welcher auch als *Destruction-*Komplex bezeichnet, besteht aus verschiedenen Proteinen, wobei zu den wichtigsten Axin, die Proteinkinase GSK-3 und das Tumorsuppressorprotein APC gehören. Wenn beta-Catenin in diesem Komplex vorliegt, kann es von beta-TrCP gebunden und ubiquitiniert werden; diese Ubiquitinierung führt dazu, dass beta-Catenin im Proteasom abgebaut wird. Wenn aber "Wnt" an seinen Rezeptor "*Frizzled*" und den Co-Rezeptor "LRP" bindet, wird das Protein "DVL" aktiviert, welches den *Destruction*-Komplex inhibiert; beta-Catenin wird freigesetzt und kann nicht mehr abgebaut werden, und es akkumuliert und gelangt auch in den Zellkern, wo es mit anderen Proteinen zusammen an einen Transkriptionsfaktor bindet, welcher auf diese Weise aktiviert wird, was wiederum in der Transkription verschiedener Gene (z. B. Cyclin D1 und MYC) resultiert.

Denn in diesem Zusammenhang hat die Anmelderin in völlig überraschender Weise gefunden, dass die relevanten Formen bzw. Ausbildungen der zugrundeliegenden Erkrankung (d. h. Nasenpolypen [*Polyposis nasi et sinuum*]) im Zusammenhang mit dem in Rede stehenden Wnt-Signalweg, insbesondere Wnt/beta-Catenin-Signaltransduktionsweg, stehen, und zwar insbesondere insofern, als die relevanten Formen der Erkrankung (d. h. Nasenpolypen) im Zusammenhang mit einer Aktivierung des in Rede stehenden Signalwegs stehen und hierdurch induziert werden, und zwar insbesondere insofern, als die Erkrankung in ihrem Verlauf in für den Patienten nachteiliger Weise beschleunigt bzw. begünstigt werden.

Insbesondere hat die Anmelderin dabei völlig überraschend gefunden, dass das im Rahmen der vorliegenden Erfindung als Wirksubstanz eingesetzte Cineol, insbesondere 1,8-Cineol, imstande ist, den Wnt-Signalweg, insbesondere Wnt/beta-Catenin-Signaltransduktionsweg, zu beeinflussen und insbesondere zu inhibieren, was mit einer verringerten Zellproliferation in Bezug auf die in Rede stehenden relevanten Formen der Erkrankung (d. h. Nasenpolypen) und somit mit einer günstigeren Verlaufsprognose für den betroffenen Patienten einhergeht. Mit anderen Worten wurde im Rahmen der vorliegenden Erfindung erstmalig und in völlig überraschender Weise gefunden, dass Cineol, insbesondere 1,8-Cineol, als Inhibitor bzw. Suppressor des Wnt-Signalwegs, insbesondere Wnt/beta-Catenin-Signaltransduktionsweg, fungiert, was zu der entsprechenden pharmakologischen Wirkeffizienz hinsichtlich der zugrundeliegenden Erkrankung (d. h. Nasenpolypen) führt.

In diesem Zusammenhang haben seitens der Anmelderin durchgeführte Untersuchungen gezeigt, dass Cineol, insbesondere 1,8-Cineol, zu einer Inhibition des Wnt/beta-Catenin-Signalwegs, führt, wobei - ohne sich auf diese Theorie beschränken zu wollen - insbesondere in Gegenwart von Cineol eine Abnahme von aktivem beta-Catenin, bei welchem es sich um ein zentrales transkriptionelles Aktivatorprotein des zugrundeliegenden Signalwegs handelt, vorliegt. In diesem Zusammenhang führt - gleichermaßen ohne sich auf diese Theorie beschränken zu wollen - Cineol bzw. 1,8-Cineol zu einem geringeren Level bzw. Gehalt an phosphorylierter GSK-3-Proteinkinase, was zu einer verstärkten Aktivität von GSK-3 und somit zu einer verstärkten Degradation von beta-Catenin führt, einhergehend mit einer Inhibierung bzw. Inaktivierung des zugrundeliegenden Wnt/beta-Catenin-Signalwegs.

Bei GSK-3 handelt es sich um eine konstitutiv aktive Proteinkinase, welche insbesondere durch die Phosphorylierung an Serin-9 (GSK-3-alpha) oder Serin-21 (GSK-3-beta) inhibiert wird. Die Verringerung der Phosphorylierung von GSK-3 führt im Rahmen der dem Wnt/beta-Catenin-Signalweg zugrundeliegenden Signalkaskade somit zu einer Aktivierung von GSK-3 als konstitutiv aktive Proteinkinase, was wiederum zu einer verstärkten Phosphorylierung und damit Inaktivierung bzw. Degradation von beta-Catenin führt. In der Folge resultiert die durch Cineol induzierte bzw. hervorgerufene Inaktivierung des Wnt/beta-Catenin-Signalwegs in Bezug auf die zu behandelnden Erkrankung (d. h. Nasenpolypen) zu einer verringerten Zellproliferation und somit zu einer Suppression bzw. Heilung oder Abschwächung im Hinblick auf die zugrundeliegende Erkrankung (d. h. Nasenpolypen). Hierzu kann auch auf die nachfolgenden Ausführungen verwiesen werden.

Darüber hinaus führt die erfindungsgemäße Verwendung von Cineol im Hinblick auf die zugrundeliegende Erkrankung (d. h. Nasenpolypen) - gleichermaßen ohne sich auf diese Theorie beschränken zu wollen - auch zu einer verringerten endolysomalen Lokalisation von GSK-3, was dessen verringerte Inhibition infolge der Behandlung mit Cineol unterstützt und was wiederum zu einer weiteren Inaktivierung von beta-Catenin führt.

Das erfindungsgemäß eingesetzte Cineol führt somit im Hinblick auf den zugrundeliegenden Wnt/beta-Catenin-Signalweg sozusagen in zweifacher Weise zu einer entsprechenden Inhibition der zugrundeliegenden Signalkaskade, einhergehend mit der zuvor angeführten Wirkeffizienz in Bezug auf die zu behandelnden Erkrankung (Nasenpolypen).

Die Anmelderin hat gleichermaßen völlig überraschend gefunden, dass im Hinblick auf die zugrundeliegende Erkrankung (d. h. Nasenpolypen) - gleichermaßen ohne sich auf diese Theorie beschränken zu wollen - die Anwendung von Cineol zu einer Verringerung der Aktivität bzw. Verringerung der Expression mindestens eines insbesondere mit dem Wnt/beta-Catenin-Signalweg im Zusammenhang stehenden Gens, vorzugsweise Wnt-Gens (synonym auch als WNT-Gen bezeichnet), führt. In diesem Zusammenhang fungiert das Cineol insbesondere als Regulator, vorzugsweise Inhibitor und/oder Suppressor, für die in Rede stehenden Gene bzw. Genprodukte, insbesondere Proteine, insbesondere für mindestens ein Wnt-Gen und/oder Wnt-Protein (synonym auch als WNT-Protein bezeichnet). Im Allgemeinen kann die Regulation, insbesondere Inhibition und/oder Suppression, auf Gen-, Transkriptions-, Translations- und/oder Proteinebene erfolgen. In diesem Zusammenhang hat die Anmelderin völlig überraschend gefunden, dass eine verringerte Aktivität des Wnt/beta-Catenin-Signalwegs insbesondere zu einer verringerten zellulären Progression im Hinblick auf die zugrundeliegende Erkrankung (d. h. Nasenpolypen) führt.

Zusammenfassend wurde erfindungsgemäß somit erstmals gefunden, dass Cineol, insbesondere 1,8-Cineol, in der Lage ist, die Wnt/beta-Catenin-Aktivität mit pharmakologischer Relevanz für die zugrundeliegende Erkrankung (Nasenpolypen) zu inhibieren. Erfindungsgemäß wird somit erstmalig ein neuer pharmakologischer Mechanismus des natürlichen Wirkstoffs Cineol aufgezeigt. Der für Cineol aufgefundene Wirkmechanismus mit der einhergehenden Wirkeffizienz für die zugrundeliegende relevante Erkrankung (d. h. Nasenpolypen) werden durch die seitens der Anmelderin durchgeführten Studien und Untersuchungen in eindrucksvoller Weise belegt, wie sie nachfolgend noch im Rahmen der Ausführungsbeispiele ausführlich referiert werden.

Gemäß einer weiteren besonderen Ausführungsform der vorliegenden Erfindung wird die erfindungsgemäße Zusammensetzung und/oder das Cineol zur prophylaktischen und/oder therapeutischen Behandlung von mit dem Wnt-Signalweg, insbesondere Wnt/beta-Catenin-Signaltransduktionsweg, in Zusammenhang stehenden rezidivierenden Formen von Nasenpolypen (*Polyposis nasi et sinuum*), vorzugsweise zur prophylaktischen und/oder therapeutischen Behandlung von über den Wnt-Signalweg, insbesondere Wnt/beta-Catenin-Signaltransduktionsweg, induzierten und/oder begünstigten rezidivierenden Formen von Nasenpolypen (*Polyposis nasi et sinuum*), eingesetzt.

Denn, wie nachfolgend noch ausgeführt wird, reduziert bzw. minimiert der Einsatz von Cineol, insbesondere 1,8-Cineol, im Rahmen des erfindungsgemäßen Therapiekonzepts die Bildung von Rezidiven in wirksamer Weise.

Gemäß einer wiederum besonderen Ausführungsform der vorliegenden Erfindung wird die erfindungsgemäße Zusammensetzung und/oder das Cineol zur prophylaktischen und/oder therapeutischen Behandlung von postoperativen Zuständen eingesetzt.

Insbesondere kann in diesem Zusammenhang die erfindungsgemäße Zusammensetzung bzw. das Cineol als Substitut oder Co-Medikation zu Kortikosteroiden eingesetzt werden, wobei durch den Einsatz von Cineol die Rezidivbildung wirksam verhindert bzw. zumindest minimiert werden kann.

Insbesondere kann die erfindungsgemäße Zusammensetzung und/oder das Cineol zur prophylaktischen und/oder therapeutischen Behandlung von Nasenpolypen (*Polyposis nasi et sinuum*), insbesondere rezidivierenden Formen, nach vorangehender chirurgischer Entfernung der Nasenpolypen eingesetzt werden.

Gemäß einer wiederum besonderen Ausführungsform der vorliegenden Erfindung kann die erfindungsgemäße Zusammensetzung und/oder das Cineol zur Verringerung des Risikos oder Wahrscheinlichkeit der Rezidivbildung, insbesondere bei postoperativen Zuständen und/oder Zuständen nach vorangehender chirurgischer Entfernung der Nasenpolypen, eingesetzt werden.

Insbesondere kann die erfindungsgemäße Zusammensetzung und/oder das Cineol zur Rezidivprophylaxe, insbesondere bei postoperativen Zuständen und/oder Zuständen nach vorangehender chirurgischer Entfernung der Nasenpolypen, eingesetzt werden.

Erfindungsgemäß bevorzugt ist es, wenn die erfindungsgemäße Zusammensetzung das Cineol als Reinstoff, insbesondere frei von anderen Terpenen, enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol. Auf diese Weise lassen sich unerwünschte Nebenwirkungen infolge des etwaigen Vorhandenseins weiterer Inhaltsstoffe, insbesondere Verunreinigungen, wirksam verhindern bzw. ausschließen.

Mit anderen Worten ist es erfindungsgemäß bevorzugt, wenn im Rahmen der erfindungsgemäßen Zusammensetzung das Cineol als Reinstoff vorliegt und/oder eingesetzt wird bzw. wenn das Cineol frei von anderen Terpenen ist und/oder keine anderen Terpene enthält.

Im Rahmen der vorliegenden Erfindung ist es somit bevorzugt, wenn das eingesetzte Cineol eine Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, aufweist.

Gemäß einer besonders bevorzugten Ausführungsform enthält die erfindungsgemäße Zusammensetzung das Cineol als 1,8-Cineol bzw. liegt das Cineol als 1,8-Cineol vor.

Gemäß einer besonderen Ausführungsform kann das Cineol in liposomenumgebener und/oder liposomal verpackter Form vorliegen. Hierdurch wird eine besondere Wirkeffizienz erreicht und das Cineol in besonderer Weise geschützt.

Bevorzugt ist es, wenn die erfindungsgemäße Zusammensetzung das Cineol als alleinigen Wirkstoff, insbesondere als alleinigen pharmazeutischen Wirkstoff, enthält und/oder wenn das Cineol als alleiniger Wirkstoff, insbesondere als alleiniger pharmazeutischer Wirkstoff, eingesetzt wird. Auf diese Weise können unerwünschte Neben- und Wechselwirkungen vermieden werden.

Gemäß einer wiederum besonderen Ausführungsform der vorliegenden Erfindung kann es vorgesehen sein, dass die erfindungsgemäße Zusammensetzung keine nichtsteroidalen Entzündungshemmer (NSAID) enthält und/oder frei ist von nichtsteroidalen Entzündungshemmern (NSAID) und/oder dass das Cineol ohne und/oder in Abwesenheit von nichtsteroidalen Entzündungshemmern (NSAID) eingesetzt und/oder verabreicht wird. Auf diese Weise können unerwünschte NSAID-Nebenwirkungen und nicht vorhersehbare Wechselwirkungen mit dem Cineol vermieden werden.

Im Rahmen der vorliegenden Erfindung enthält die erfindungsgemäße Zusammensetzung das Cineol in wirksamen, insbesondere pharmazeutisch wirksamen Mengen.

Die Menge an Cineol in der erfindungsgemäßen Zusammensetzung kann, insbesondere in Abhängigkeit von der Therapieform (z. B. topisch oder systemisch) und der Schwere und Eigenart der zu behandelnden Erkrankung, in weiten Grenzen variieren. Insbesondere kann es erfindungsgemäß vorgesehen sein, wenn die erfindungsgemäße Zusammensetzung das Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält. Auf diese Weise werden eine gute Wirkeffizienz einerseits und eine gute Verträglichkeit andererseits gewährleistet. Dennoch kann es vorgesehen sein, von den genannten Werten abzuweichen, ohne dass der Rahmen der vorliegenden Erfindung verlassen ist; dies liegt im fachmännischen Ermessen in jedem Einzelfall.

Insbesondere kann es erfindungsgemäß vorgesehen sein, wenn die erfindungsgemäße Zusammensetzung das Cineol, bezogen auf die Zusammensetzung, in relativen Mengen von mindestens 0,0001 Gew.-%, insbesondere von mindestens 0,001 Gew.-%, vorzugsweise von mindestens 0,005 Gew.-%, bevorzugt von mindestens 0,01 Gew.-%, besonders bevorzugt von mindestens 0,05 Gew.-%, ganz besonders bevorzugt von mindestens 0,1 Gew.-%, enthält.

Weiterhin kann es erfindungsgemäß insbesondere vorgesehen sein, wenn die erfindungsgemäße Zusammensetzung das Cineol, bezogen auf die Zusammensetzung, in relativen Mengen von höchstens 80 Gew.-%, insbesondere von höchstens 75 Gew.-%, vorzugsweise von höchstens 70 Gew.-%, bevorzugt von höchstens 60 Gew.-%, besonders bevorzugt von höchstens 55 Gew.-%, ganz besonders bevorzugt von höchstens 50 Gew.-%, enthält.

Üblicherweise ist es vorgesehen, dass die erfindungsgemäße Zusammensetzung das Cineol zusammen mit mindestens einem physiologisch unbedenklichen Träger (Exzipienten) enthält bzw. dass das Cineol zusammen mit mindestens einem physiologisch unbedenklichen Träger (Exzipienten) eingesetzt wird. Dabei können grundsätzlich alle physiologisch unbedenklichen Träger (Exzipienten) eingesetzt werden. Insbesondere ist es bevorzugt, wenn der eingesetzte Träger (Exzipient) mit 1,8-Cineol mischbar und/oder hierin löslich ist und/oder wenn der Träger (Exzipient) bei 20 °C und bei Atmosphärendruck im flüssigen oder festen Aggregatzustand vorliegt. Beispiele für mit 1,8-Cineol mischbaren und/oder hierin löslichen, insbesondere bei 20 °C und Atmosphärendruck flüssigen Trägern (Exzipienten) sind beispielsweise Verbindungen aus der Gruppe von fetten Ölen, bevorzugt Triglyceriden, besonders bevorzugt mittelkettigen Triglyceriden (*Medium Chain Triglycerides*, MCT), ganz besonders bevorzugt Triglyceriden mit C₆-C₁₂-Fettsäureresten.

Im Rahmen der vorliegenden Erfindung kann es vorgesehen sein, dass die erfindungsgemäße Zusammensetzung entweder topisch oder aber systemisch appliziert wird bzw. dass die erfindungsgemäße Zusammensetzung zur topischen oder systemischen Applikation hergerichtet ist.

Mit anderen Worten bedeutet dies, dass im Rahmen der vorliegenden Erfindung das Cineol topisch oder systemisch appliziert wird bzw. das Cineol zur topischen oder systemischen Applikation hergerichtet ist.

Wie bereits zuvor ausgeführt, kann die erfindungsgemäße Zusammensetzung bzw. das Cineol im Rahmen der erfindungsgemäßen Therapie grundsätzlich topisch (z. B. intranasal und/oder inhalativ) und/oder topisch (z. B. systemisch) appliziert werden, und zwar in Abhängigkeit von der individuellen Therapie bzw. von der zu therapierenden Form, der Schwere der Erkrankung, dem jeweiligen Patienten etc.

Erfindungsgemäß ist es also vorgesehen, dass die erfindungsgemäße Zusammensetzung und/oder das Cineol topisch, insbesondere intranasal oder inhalativ, appliziert wird und/oder dass die erfindungsgemäße Zusammensetzung und/oder das Cineol zur topischen, insbesondere intranasalen oder inhalativen Applikation hergerichtet ist.

Im Fall der topischen Anwendung kann die erfindungsgemäße Zusammensetzung und/oder das Cineol in Form einer intranasal zu verabreichenden Darreichungsform appliziert werden. Dies bedeutet, dass die erfindungsgemäße Zusammensetzung und/oder das Cineol in einer intranasal zu verabreichenden Darreichungsform hergerichtet sein kann. Beispielsweise kann die erfindungsgemäße Zusammensetzung in Form von Nasensprays, Nasentropfen, Nasengelen, Nasensalben, Nasenspülungen, Nasencremes, Inhalaten oder dergleichen, bevorzugt Nasensprays, appliziert werden. Dies bedeutet, dass die erfindungsgemäße Zusammensetzung und/oder das Cineol in Form von Nasensprays, Nasentropfen, Nasengelen, Nasensalben, Nasenspülungen, Nasencremes, Inhalaten oder dergleichen, bevorzugt Nasensprays, hergerichtet sein kann.

Gemäß einer weiteren Variante der vorliegenden Erfindung kann aber - wie zuvor ausgeführt - die Zusammensetzung und/oder das Cineol auch systemisch, insbesondere peroral oder parenteral (z. B. intravenös, intraarteriell, intramuskulär, subkutan etc.; beispielsweise in Form einer parenteralen Darreichungsform, wie Injektion, Infusion etc.), vorzugsweise peroral, appliziert werden. D. h. die erfindungsgemäße Zusammensetzung und/oder das Cineol können in diesem Fall zur systemischen, insbesondere peroralen oder parenteralen, vorzugsweise peroralen Applikation hergerichtet sein.

Dabei kann die erfindungsgemäße Zusammensetzung und/oder das Cineol insbesondere in Form einer peroral zu verabreichenden Darreichungsform appliziert werden, d. h. die erfindungsgemäße Zusammensetzung und/oder das Cineol können in diesem Fall in einer peroral zu verabreichenden Darreichungsform hergerichtet sein.

Bei dieser Ausführungsform kann die erfindungsgemäße Zusammensetzung und/oder das Cineol insbesondere als magensaftresistente, aber dünndarmlösliche systemische Darreichungsform, bevorzugt als Kapsel, Dragee, Pille, Tablette oder dergleichen, appliziert werden, d. h. die erfindungsgemäße Zusammensetzung und/oder das Cineol können bei dieser Ausführungsform als magensaftresistente, aber dünndarmlösliche systemische Darreichungsform, bevorzugt als Kapsel, Dragee, Pille, Tablette oder dergleichen, hergerichtet sein. Des Weiteren kann bei dieser Ausführungsform die erfindungsgemäße Zusammensetzung und/oder das Cineol insbesondere auch als Injektion, Infusion oder dergleichen hergerichtet sein.

Insbesondere in Abhängigkeit von der Art der Darreichung (d. h. systemisch und/oder topisch) und der Schwere und Eigenart der zu behandelnden Erkrankung sowie weiterer therapeutischer Faktoren und Gegebenheiten kann die Tagesdosis des applizierten Cineols in weiten Bereichen variieren. Insbesondere kann das Cineol mit einer Tagesdosis im Bereich von 0,1 bis 5.000 mg/diem, insbesondere im Bereich von 1 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht werden. Dies bedeutet, dass die erfindungsgemäße Zusammensetzung und/oder das Cineol zur Verabreichung mit einer Tagesdosis im Bereich von 0,1 bis 5.000 mg/diem, insbesondere im Bereich von 1 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, hergerichtet sein kann. Dennoch kann es grundsätzlich vorgesehen sein, von den genannten Werten abzuweichen, ohne dass der Rahmen der vorliegenden Erfindung verlassen ist. Dies liegt im Ermessen des Fachmanns in jedem Einzelfall.

Bei systemischer Anwendung können die eingesetzten Mengen an Cineol gleichermaßen in weiten Bereichen variieren. Insbesondere kann es vorgesehen sein, dass das Cineol systemisch mit einer Tagesdosis im Bereich von 10 bis 5.000 mg/diem, insbesondere im Bereich von 50 bis 3.000 mg/diem, vorzugsweise im Bereich von 100 bis 2.500 mg/diem, bevorzugt im Bereich von 150 bis 2.000 mg/diem, besonders bevorzugt im Bereich von 200 bis 1.500 mg/diem, verabreicht wird und/oder dass die erfindungsgemäße Zusammensetzung und/oder das Cineol zur Verabreichung mit einer Tagesdosis im Bereich von 10 bis 5.000 mg/diem, insbesondere im Bereich von 50 bis 3.000 mg/diem, vorzugsweise im Bereich von 100 bis 2.500 mg/diem, bevorzugt im Bereich von 150 bis 2.000 mg/diem, besonders bevorzugt im Bereich von 200 bis 1.500 mg/diem, hergerichtet sein kann. Grundsätzlich kann es jedoch vorgesehen sein, von den genannten Werten abzuweichen, ohne dass der Rahmen der vorliegenden Erfindung verlassen ist (was im Ermessen des Fachmanns in jedem Einzelfall liegt).

Auch bei topischer Anwendung können die eingesetzten Cineoldosen in weiten Bereichen variieren. Insbesondere kann bei topischer Anwendung vorgesehen sein, dass das Cineol topisch mit einer Tagesdosis im Bereich von 0,1 bis 2.000 mg/diem, insbesondere im Bereich von 0,5 bis 1.500 mg/diem, vorzugsweise im Bereich von 1 bis 1.000 mg/diem, bevorzugt im Bereich von 2 bis 1.500 mg/diem, besonders bevorzugt im Bereich von 5 bis 1.000 mg/diem, verabreicht wird bzw. dass die Zusammensetzung und/oder das Cineol zur Verabreichung mit einer Tagesdosis im Bereich von 0,1 bis 2.000 mg/diem, insbesondere im Bereich von 0,5 bis 1.500 mg/diem, vorzugsweise im Bereich von 1 bis 1.000 mg/diem, bevorzugt im Bereich von 2 bis 1.500 mg/diem, besonders bevorzugt im Bereich von 5 bis 1.000 mg/diem, hergerichtet sein kann. Grundsätzlich kann es jedoch vorgesehen sein, von den genannten Werten abzuweichen, ohne dass der Rahmen der vorliegenden Erfindung verlassen ist (was im Ermessen des Fachmanns in jedem Einzelfall liegt).

Üblicherweise kann die erfindungsgemäße Zusammensetzung außerdem mindestens einen weiteren Inhaltsstoff, insbesondere einen weiteren Inhaltsstoff, insbesondere mindestens einen Hilfsstoff und/oder ein Additiv, enthalten. Dies bedeutet, dass das erfindungsgemäße Cineol mit mindestens einem weiteren Inhaltsstoff, insbesondere mindestens einem Hilfsstoff und/oder Additiv, eingesetzt wird. In diesem Zusammenhang kann der mindestens eine weitere Inhaltsstoff, insbesondere Hilfsstoff und/oder Additiv, insbesondere ausgewählt sein aus der Gruppe von Verarbeitungshilfsstoffen, Stabilisatoren, Emulgatoren, Antioxidantien, Feuchthaltemitteln, Verdickungsmitteln, Desinfektionsmitteln, pH-Stellmitteln, pH-Puffersubstanzen, Konservierungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Gemäß einer besonderen Ausführungsform der vorliegenden Erfindung kann die erfindungsgemäße Zusammensetzung und/oder das Cineol in Ergänzung, bevorzugt in Kombination, mit Kortikosteroiden, insbesondere systemischen oder topischen Kortikosteroiden, eingesetzt werden. Dies bedeutet, das bei diesen Ausführungsformen die erfindungsgemäße Zusammensetzung und/oder das Cineol als Ergänzungstherapeutikum (Co-Therapeutikum), bevorzugt als Kombinationstherapeutikum, mit Kortikosteroiden, insbesondere systemischen oder topischen Kortikosteroiden, eingesetzt werden kann.

Im Rahmen der vorliegenden Erfindung kann es somit vorgesehen sein, dass die erfindungsgemäße Zusammensetzung und/oder das Cineol, insbesondere in Co-Therapie oder in Kombination mit Kortikosteroiden, zur Reduktion des Bedarfs oder zum Ersatz von Kortikosteroiden bei der prophylaktischen und/oder therapeutischen Behandlung von Nasenpolypen (*Polyposis nasi et sinuum*) eingesetzt werden kann.

Insbesondere kann es im Rahmen der vorliegenden Erfindung vorgesehen sein, dass die erfindungsgemäße Zusammensetzung und/oder das Cineol, insbesondere in Co-Therapie oder in Kombination mit Kortikosteroiden, zur Wirkungssteigerung und/oder Dosisreduktion von Kortikosteroiden bei der prophylaktischen und/oder therapeutischen Behandlung von Nasenpolypen (*Polyposis nasi et sinuum*) eingesetzt werden kann.

Wie zuvor dargelegt, kann im Rahmen der Co-Therapie mit Kortikosteroiden deren Wirkung gesteigert und deren Dosis verringert werden. Folglich kann insbesondere bei Co-Therapie mit Kortikosteroiden vorgesehen sein, dass die erfindungsgemäße Zusammensetzung und/oder das Cineol, insbesondere in Co-Therapie oder in Kombination mit Kortikosteroiden, zur Verringerung oder Vermeidung von Nebenwirkungen von Kortikosteroiden bei der prophylaktischen und/oder therapeutischen Behandlung von Nasenpolypen (*Polyposis nasi et sinuum*) eingesetzt wird.

Gemäß einer weiteren besonderen Ausführungsform der vorliegenden Erfindung kann es zudem vorgesehen sein, dass die erfindungsgemäße Zusammensetzung und/oder das Cineol nicht in Ergänzung, insbesondere nicht in Kombination, mit nichtsteroidalen Entzündungshemmern (NSAID) eingesetzt wird und/oder dass die Zusammensetzung und/oder das Cineol nicht als Ergänzungstherapeutikum (Co-Therapeutikum), insbesondere nicht als Kombinationstherapeutikum, mit nichtsteroidalen Entzündungshemmern (NSAID) eingesetzt wird. Auf diese Weise werden die unerwünschten Nebenwirkungen von NSAID in wirksamer Weise verhindert (zumal deren Verwendung im Rahmen des erfindungsgemäßen Therapiekonzepts nicht erforderlich ist).

Mit anderen Worten ist es auf Basis der erfindungsgemäßen Zusammensetzung vorgesehen, Cineol, insbesondere 1,8-Cineol, bei der prophylaktischen und/oder therapeutischen Behandlung von Nasenpolypen (*Polyposis nasi et sinuum*) zu verwenden.

Die vorliegende Erfindung betrifft somit die Verwendung einer erfindungsgemäßen, Cineol, vorzugsweise 1,8-Cineol, als pharmazeutischen Wirkstoff enthaltenden Zusammensetzung, insbesondere pharmazeutischen Zusammensetzung, wie sie zuvor beschrieben bzw. definiert worden ist, zur prophylaktischen und/oder therapeutischen Behandlung von Nasenpolypen (*Polyposis nasi et sinuum*) und/oder zur Herstellung eines Arzneimittels zur prophylaktischen und/oder therapeutischen Behandlung von Nasenpolypen (*Polyposis nasi et sinuum*).

Erfindungsgemäß ist es somit möglich, Cineol, vorzugsweise 1,8-Cineol, als pharmazeutischen Wirkstoff zur prophylaktischen und/oder therapeutischen Behandlung von Nasenpolypen (*Polyposis nasi et sinuum*) und/oder zur Herstellung eines Arzneimittels zur prophylaktischen und/oder therapeutischen Behandlung von Nasenpolypen (*Polyposis nasi et sinuum*) zu verwenden.

Ein wiederum weiterer Gegenstand der vorliegenden Erfindung - gemäß einem **zweiten** Aspekt der vorliegenden Erfindung - ist zudem ein Arzneimittel zur prophylaktischen und/oder therapeutischen Behandlung von Nasenpolypen (*Polyposis nasi et sinuum*) bzw. zur Verwendung bei der prophylaktischen und/oder therapeutischen Behandlung von Nasenpolypen (*Polyposis nasi et sinuum*), wobei das Arzneimittel als Wirkstoff, insbesondere pharmazeutischen Wirkstoff, Cineol, vorzugsweise 1,8-Cineol, enthält.

Der Begriff des Arzneimittels (synonym auch als Pharmazeutikum, Medikament, Therapeutikum oder dergleichen bezeichnet), wie er im Rahmen der vorliegenden Erfindung verwendet wird, ist sehr umfänglich zu verstehen und umfasst nicht nur Arzneimittel bzw. Pharmazeutika als solche (d. h. in arzneimittelrechtlicher Hinsicht), sondern vor allem auch sogenannte Medizinprodukte und darüber hinaus aber auch Homöopathika und Nahrungsergänzungsmittel. Mit anderen Worten kann also die erfindungsgemäße cineolhaltige Zusammensetzung nach der Erfindung in Form eines Arzneimittels (Pharmazeutikums), Medizinprodukts, Homöopathikums, Nahrungsergänzungsmittels oder dergleichen vorliegen.

Für weitergehende Einzelheiten zu diesem Erfindungsaspekt kann auf die vorangehenden Ausführungen zu den übrigen Erfindungsaspekten verwiesen werden, welche in Bezug auf diesen Erfindungsaspekt entsprechend gelten.

Des Weiteren ist ein wiederum weiterer Gegenstand der vorliegenden Erfindung - gemäß einem **dritten** Aspekt der vorliegenden Erfindung - eine Wirkstoffkombination, insbesondere in Form eines Kit (Kit-of-parts), zur Verwendung bei der prophylaktischen und/oder therapeutischen Behandlung von Nasenpolypen (*Polyposis nasi et sinuum*),
wobei die Wirkstoffkombination als räumlich getrennte, aber funktional zusammenhängende und/oder zur Co-Medikation oder Kombinationstherapie bestimmte Komponenten
(a) Cineol, insbesondere 1,8-Cineol, bevorzugt in Form einer das Cineol enthaltenden, topisch oder systemisch zu applizierenden pharmazeutischen Zusammensetzung, einerseits und
(b) mindestens ein Kortikosteroid, bevorzugt in Form einer das Kortikosteroid enthaltenden, topisch oder systemisch zu applizierenden pharmazeutischen Zusammensetzung,
umfasst.

Für weitergehende Einzelheiten zu diesem Erfindungsaspekt kann auf die vorangehenden Ausführungen zu den übrigen Erfindungsaspekten verwiesen werden, welche in Bezug auf diesen Erfindungsaspekt entsprechend gelten.

Im Rahmen der vorliegenden Erfindung hat sich gezeigt, dass Cineol, insbesondere 1,8-Cineol, zur Regulation, vorzugsweise Inhibition und/oder Suppression, des Wnt-Signalwegs, insbesondere Wnt/beta-Catenin-Signaltransduktionswegs, vorzugsweise zur Regulation und/oder Verringerung der Phosphorylierung der GSK-3-Proteinkinase, insbesondere an Serin-9 (GSK-3-alpha) und/oder Serin-21 (GSK-3-beta), im Rahmen des Wnt-Signalwegs bzw. zur Aktivierung der GSK-3-Proteinkinase und/oder zur Deaktivierung und/oder Phosphorylierung von beta-Catenin im Rahmen des Wnt-Signalwegs, zur Verwendung bei der prophylaktischen und/oder therapeutischen Behandlung von Nasenpolypen (*Polyposis nasi et sinuum*), vorzugsweise rezidivierenden Formen von Nasenpolypen (*Polyposis nasi et sinuum*), verwendet werden kann.

Auf diese Weise lassen sich mit dem erfindungsgemäßen Therapiekonzept sehr spezifisch bzw. selektiv spezielle Krankheitszustände behandeln, welche herkömmlichen Therapieformen nicht ohne Weiteres zugänglich sind, zumindest nicht mit einer derart spezifischen bzw. selektiven Wirkweise.

Schließlich ist ein wiederum weiterer Gegenstand der vorliegenden Erfindung - gemäß einem **vierten** Aspekt der vorliegenden Erfindung - Cineol, insbesondere 1,8-Cineol, zur Verwendung bei der prophylaktischen und/oder therapeutischen Behandlung von mit dem Wnt-Signalweg, insbesondere Wnt/beta-Catenin-Signaltransduktionsweg, in Zusammenhang stehenden und/oder hierdurch induzierten und/oder begünstigten Erkrankungen, insbesondere von Nasenpolypen (*Polyposis nasi et sinuum*), vorzugsweise von rezidivierenden Formen von Nasenpolypen (*Polyposis nasi et sinuum*).

Bei diesem Erfindungsaspekt wird das Cineol insbesondere als Regulator, vorzugsweise Inhibitor und/oder Suppressor, des Wnt-Signalwegs, insbesondere Wnt/beta-Catenin-Signaltransduktionswegs, vorzugsweise als Regulator und/oder Verringerer der Phosphorylierung der GSK-3-Proteinkinase im Rahmen des Wnt-Signalwegs bzw. zur Aktivierung der GSK-3-Proteinkinase und/oder als Deaktivator von beta-Catenin im Rahmen des Wnt-Signalwegs, eingesetzt.

Insbesondere kann das Cineol bei diesem Erfindungsaspekt auch als Regulator, vorzugsweise Inhibitor und/oder Suppressor, mindestens eines Wnt-Gens und/oder Wnt-Proteins eingesetzt werden.

Auch gemäß diesem Erfindungsaspekt lassen sich sehr spezifisch bzw. selektiv spezielle Krankheitszustände behandeln, welche herkömmlichen Therapieformen nicht ohne Weiteres zugänglich sind, zumindest nicht mit einer derart spezifischen bzw. selektiven Wirkweise.

Für weitergehende Einzelheiten zu diesem Erfindungsaspekt kann auf die vorangehenden Ausführungen zu den übrigen Erfindungsaspekten verwiesen werden, welche in Bezug auf diesen Erfindungsaspekt entsprechend gelten.

Weitere vorteilhafte Eigenschaften, Aspekte und Merkmale der vorliegenden Erfindung ergeben sich aus der folgenden Beschreibung von in den Figuren dargestellten Ausführungsbeispielen, wie sie auch nachfolgend noch im Detail angeführt sind. Es zeigt:

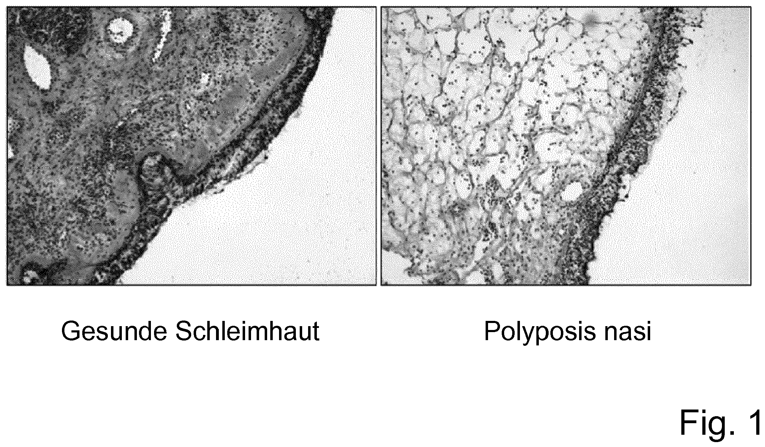

- Fig. 1: Hämatoxylin-Eosin (HE)-Färbung von Nasenpolypen und gesunder Nasenschleimhaut (Diese Färbemethode färbt basophile Strukturen blaulila und eosinophile Strukturen in hellem Pink, wohingegen hydrophobe Strukturen, wie die Membranen des Golgi-Apparats, ungefärbt bzw. klar bleiben.);

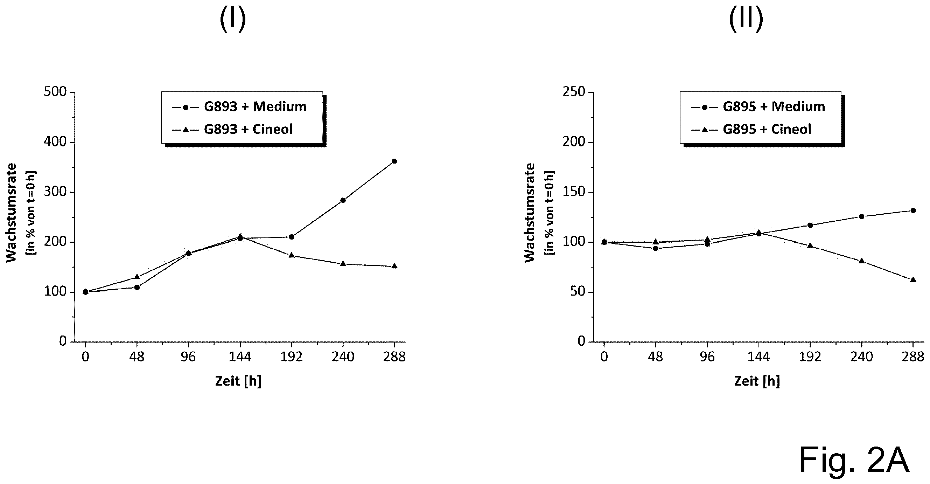

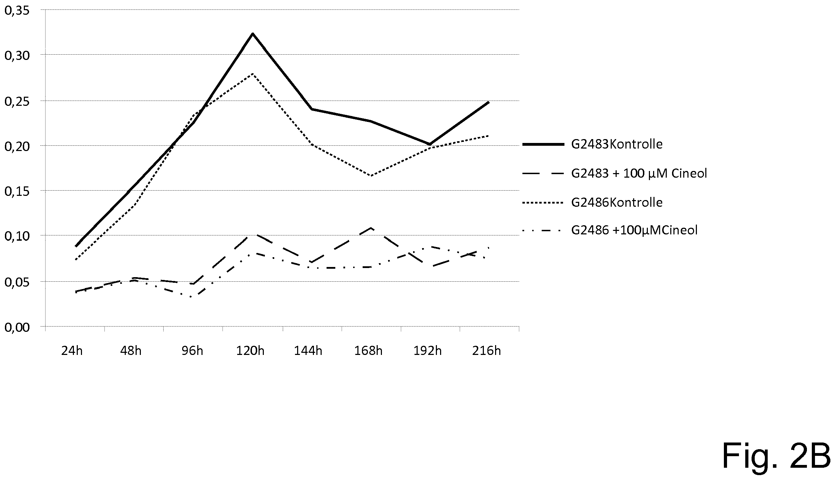

- Fig. 2: Dosisabhängige Reduktion der zellulären Progression durch 1,8-Cineol (Um zu bestimmen, ob die Behandlung mit 1,8-Cineol zu einer Inhibierung der zellulären Lebensfähigkeit von Nasenpolypen führt, wurden MTT-Tests eingesetzt. Eine stark abnehmende Proliferation von Nasenpolypen-Zellen konnte als Reaktion auf eine Inkubation mit 100 µM 1,8-Cineol nach 144 Stunden beobachtet werden (Fig. 2A, mit zwei verschiedenen Probanden I und II). Überraschenderweise wurden kultivierte Epithelzellen der Nasenschleimhaut durch Behandlung mit 1,8-Cineol nicht beeinflusst, was einen kontextabhängigen spezifischen Wirkmechanismus von 1,8-Cineol nahelegt (Fig. 2B));

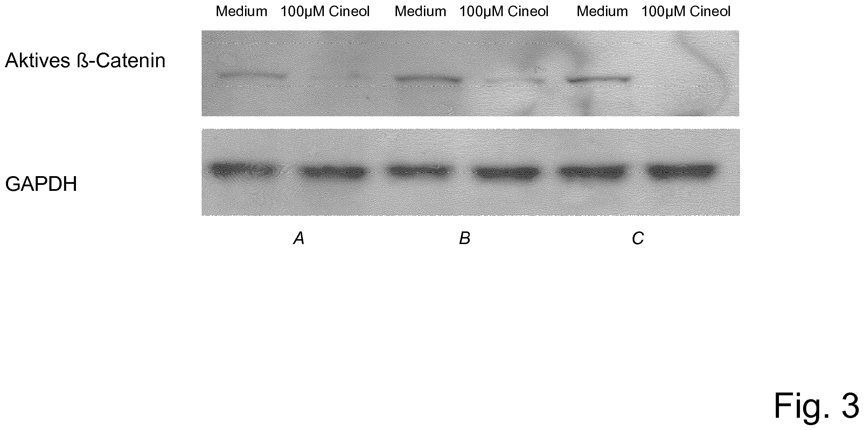

- Fig. 3: Expression von aktivem beta-Catenin auf Antwort auf Cineol (Um den Effekt einer 24 Stunden-Behandlung mit 100 µM 1,8-Cineol auf das Protein-Expressions-Level von aktivem beta-Catenin in drei verschiedenen Gewebeproben von Nasenpolypen (A, B, C) zu untersuchen, wurden Western-Blot-Analysen an Gesamtproteinen durchgeführt. GAPDH wurde als Beladungskontrolle eingesetzt.);

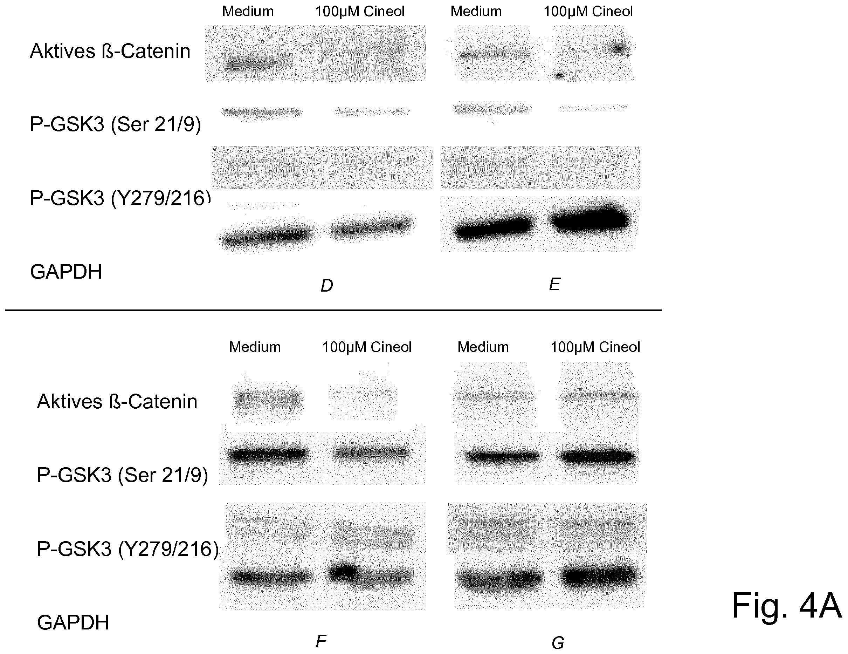

- Fig. 4A: Expression von aktivem beta-Catenin und P-GSK-3 (Serin-9/-21) als Reaktion auf Cineol (Um den Effekt einer 24-stündigen Behandlung mit 100 µM 1,8-Cineol auf die Protein-Expression-Level von aktivem beta-Catenin, Phospho-GSK-3 (Serin-9/-21) und Phospho-GSK-3 (Tyrosin-279/-216) in vier verschiedenen Proben von Nasenpolypen (D, E, F, G) zu untersuchen, wurden Western-Blot-Analysen an Gesamtproteinen durchgeführt. GAPDH wurde als Beladungskontrolle verwendet.);

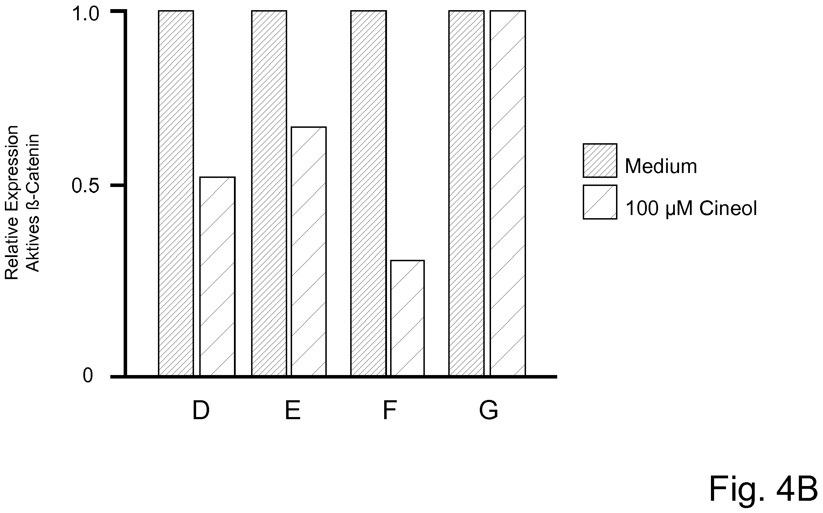

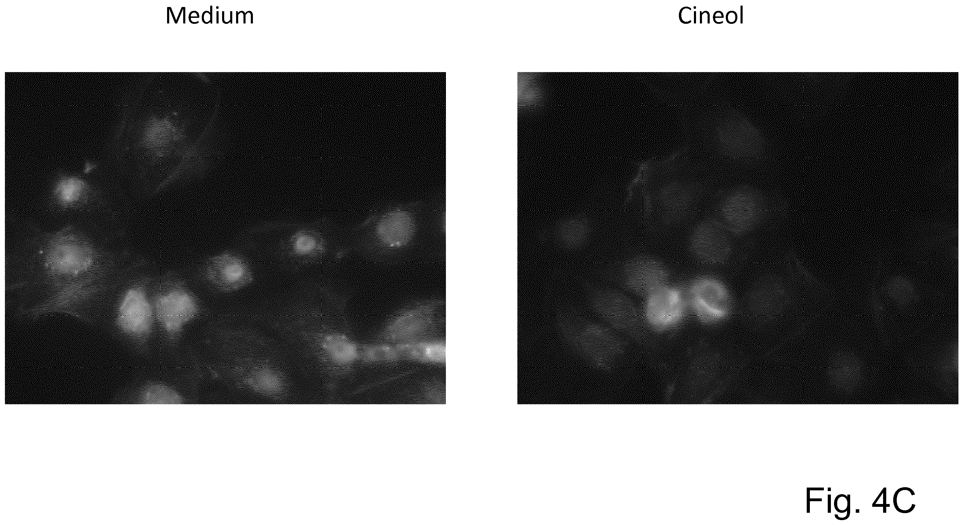

- Fig. 4B/C: Subzelluläre Lokalisation von P-GSK-3 (Serin-9/-21) als Reaktion auf Cineol (Um eine verringerte endolysosomale Lokalisierung von Phospho-GSK-3 (Serin-9/-21) als Reaktion auf 100 µM 1,8-Cineol, verglichen zum Kontrollmedium, zu untersuchen, wurden immunohistochemische Untersuchungen durchgeführt. Zellkerne wurden mit DAPI angefärbt.);

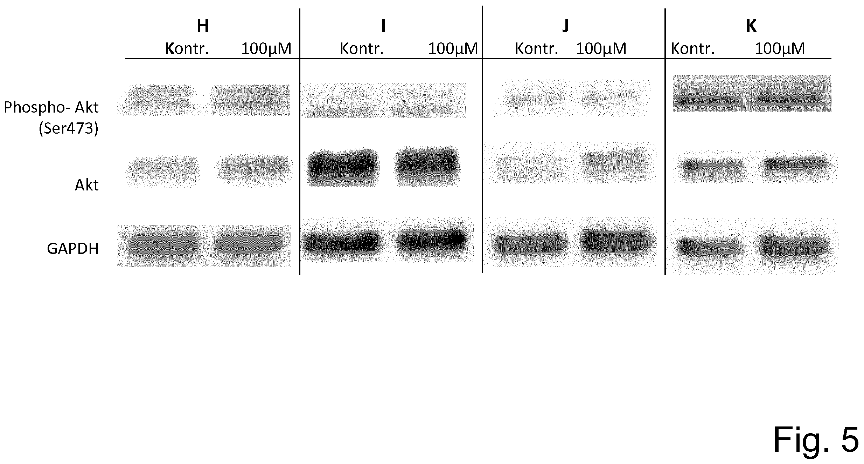

- Fig. 5: Expression von Akt und P-Akt (Serin-473) als Reaktion auf 1,8-Cineol (Western Blot-Analysen an Gesamtproteinen wurden eingesetzt, um den Effekt von 100 µM 1,8-Cineol auf die Expressions-Level von Akt und P-Akt (Threonin-308) in vier verschiedene Gewebeproben von Nasenpolypen zu zeigen (H, I, J, K). GAPDH wurde als Beladungskontrolle verwendet.);

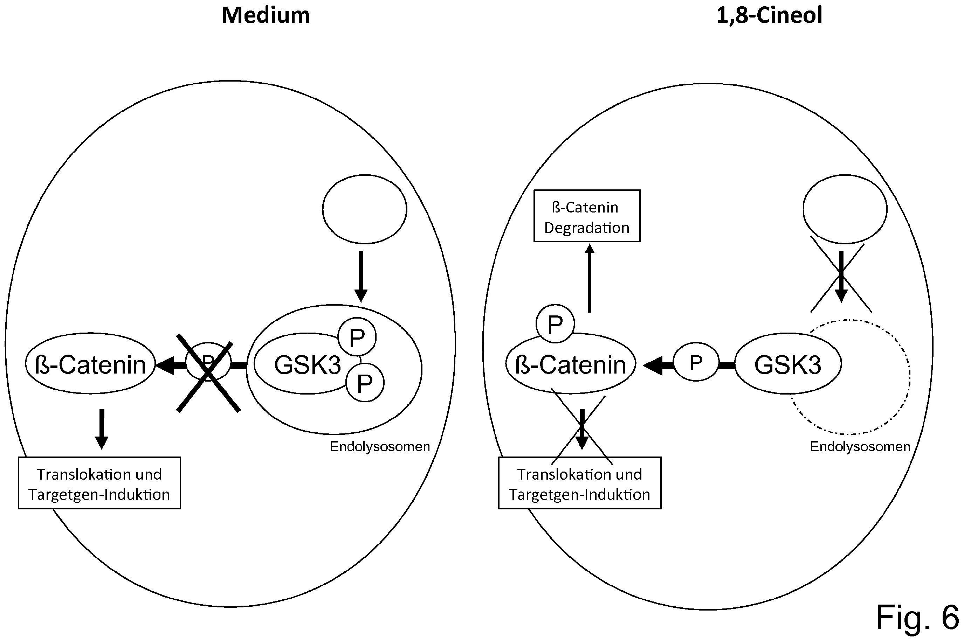

- Fig. 6: Modell der Wnt/beta-Catenin-Inhibition als Reaktion auf 1,8-Cineol (Im Rahmen dieser Arbeit hat die Anmelderin erstmals gezeigt, dass 1,8-Cineol als Inhibitor für die Wnt/beta-Catenin-Aktivität fungiert und einen starken Rückgang der zellulären Progression von Nasenpolypen bewirkt. Die Daten der Anmelderin zeigen, dass 1,8-Cineol die inhibitorische Phosphorylierung und endolysosomale Lokalisierung von GSK3-beta, bei welchem es sich um den zentralen Regulator der beta-Catenin-Aktivität handelt, beeinflusst.).

Für weitergehende Einzelheiten zu den in den Figurendarstellungen dargestellten Ausführungsformen und Ausführungsbeispielen kann zur Vermeidung unnötiger Wiederholungen auf die obigen Ausführungen sowie auf die nachfolgenden ergänzenden Ausführungen zu den Ausführungsbeispielen verwiesen werden, welche in Bezug auf die Figurendarstellung entsprechend gelten.

Zusammenfassend kann also im Rahmen der vorliegenden Erfindung erstmals ein neuartiges Therapiekonzept für die prophylaktische und/oder therapeutische Behandlung von Nasenpolypen (*Polyposis nasi et sinuum*), insbesondere auch rezidivierenden Formen, bereitgestellt werden, mit welchem die Nachteile des Standes der Technik zumindest weitgehend vermieden oder aber wenigstens abgeschwächt werden können, wobei das erfindungsgemäße Therapiekonzept gegenüber herkömmlichen Therapiemethoden insbesondere mit weniger Nebenwirkungen und/oder Unannehmlichkeiten für die betroffenen Patienten verbunden, wobei im Rahmen der vorliegenden Erfindung eine mindestens vergleichbare, bevorzugt sogar eine verbesserte Wirkeffizienz im Vergleich zu den Therapiekonzepten des Standes der Technik erreicht wird. Ziel des erfindungsgemäßen Therapiekonzepts ist insbesondere auch eine anhaltende Rezidivfreiheit bzw. zumindest eine Verlängerung des rezidivfreien Intervalls, insbesondere infolge der antientzündlichen und kortikosteroidartigen Wirkung von Cineol, insbesondere 1,8-Cineol. Auch kommt es zu einer Vermeidung oder zumindest Reduktion der bekannten Dauertherapie mit Kortikosteroiden (Glucokortikoiden), insbesondere auch bei gleichzeitig bestehendem *Asthma bronchiale.*

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. 1,8-Cineol reguliert das Phosphokinaseprofil in aus Nasenpolypen und unteren Nasenmuscheln stammenden Epithelzellen von an chronischer Rhinosinusitis leidenden Patienten

### Stichworte: 1,8-Cineol, Cineol, Phosphokinaseprofil, Nasenpolypen, chronische Rhinosinusitis, CRSwNP, chronische Rhinosinusitis mit Nasenpolypen

Neueste Untersuchungsergebnisse zeigen eine Beteiligung von Phosphokinasen an der Pathogenese von chronischer Rhinosinusitis mit Nasenpolypen (CRSwNP). So kann der Phosphorylierungsstatus von speziellen Proteinen zu einer Ausbildung von Nasenpolypen beitragen. Auf Basis dieser Ergebnisse wurden nun neue Erkenntnisse für eine potentielle Regulierung der Phosphorylierung in Nasenpolypen auf Pflanzenbasis gewonnen. Derzeit wird chronische Rhinosinusitis in chronische Rhinosinusitis mit Nasenpolypen (CRSwNP) und chronische Rhinosinusitis ohne Nasenpolypen (CRSsNP) unterteilt, wobei CRSwNP circa 1 bis 4 % der allgemeinen Bevölkerung betrifft. Es herrscht allgemeine Übereinkunft, dass alle Fälle von chronischer Rhinosinusitis (CRS) mit sinunasalen Entzündungen einhergehen, wobei CRSwNP als Th2-gesteuerter Prozess und im Gegensatz dazu CRSsNP als Th1-gesteuerter Prozess verstanden wird. Die Handhabung von CRSwNP umfasst eine Kombination von medikamentöser Therapie und chirurgischen Maßnahmen. Üblicherweise werden Kortikosteroide (Glucocorticoide) sowohl als primäre Behandlung als auch als postoperative Prophylaxe gegen Rezidive eingesetzt. Kortikosteroide (Glucocorticoide) besitzen antiinflammatorische Eigenschaften auf Basis von Protein-Protein Interaktionen, wobei jedoch längerfristige Applikationen die Nebenwirkungen verstärken.

Pflanzliche Medikationen könnten diese Nebenwirkungen reduzieren. In der Naturheilkunde werden ätherische Öle aufgrund ihrer sekretolytischen Eigenschaften zur Behandlung von Menschen eingesetzt, die klinische Verwendung wird jedoch aufgrund von Reizungen der Atemwege nach Inhalation bzw. Aufnahme eingeschränkt. Der Hauptbestandteil von Eukalyptusöl, nämlich 1,8-Cineol, wurde zur Behandlung von akuter und chronischer Bronchitis, Sinusitis und respiratorischen Infekten eingesetzt und allgemein gut vertragen. Bei dem Monoterpen 1,8-Cineol handelt es sich um ein Isoprenoid mit antiinflammatorischen Eigenschaften, welches menschlichen Isoprenoiden, wie Glucocorticoid, ähnelt. Unlängst wurden seitens der Anmelderin neue Eigenschaften des Mechanismus von 1,8-Cineol in Epithelzellen des Nasenpolypengewebes von Patienten mit chronischer Rhinosinusitis entdeckt. Im Rahmen dieser Studien hat die Anmelderin Epithelzellen aus Nasenpolypen und Gewebe der unteren Nasenmuscheln von zahlreichen Patienten isoliert und über einen Zeitraum von 24 Stunden in 100 µM 1,8-Cineol inkubiert. Die Proteine wurden isoliert und die Phosphokinasen mit Hilfe des "*Human Phospho-Kinase Antibody Array*" von R&D Systems ermittelt. Interessanterweise hat die Anmelderin im Vergleich zu Epithelzellen aus Nasenmuscheln eine veränderte Expression von Phosphokinasen in nasalen Epithelzellen aus Nasenpolypengewebe bestimmt. Selbst wenn nasale Epithelzellen aus Nasenpolypen einerseits und aus unteren Nasenmuscheln andererseits den gleichen chronischen entzündlichen Bedingungen ausgesetzt sind, sprechen sie unterschiedlich auf 1,8-Cineol an. Das Schleimhautepithel in der Nase ist ständig Umweltfaktoren, wie Allergenen, Bakterien oder Viren, ausgesetzt und dient als erste Abwehr, wobei jedoch die Ergebnisse der Anmelderin einen grundlegenden Unterschied zwischen nasalen Epithelzellen aus Nasenpolypen einerseits und aus unteren Nasenmuscheln andererseits bezüglich der Phosphorylierung von Kinasen in dem Ansprechen auf 1,8-Cineol belegen. Auf dieser Basis werden neue Perspektiven für dessen therapeutische Verwendung im Rahmen der Behandlung von Erkrankungen der Atemwege, insbesondere CRSwNP, aufgezeigt, da Cineol nachweislich eine phytotherapeutische Alternative zu Glucokortikoiden darstellt.

### 2. 1,8-Cineol inhibiert den Wnt/beta-Catenin-Signalweg und die zelluläre Proliferation in Nasenpolypen

### Stichworte: Nasenpolypen, chronische Rhinosinusitis, GSK-3, beta-Catenin, 1,8-Cineol

### Zusammenfassung

**Hintergrund:** Nasenpolypen stellen eine benigne Neoplasie der Nasenschleimhaut dar, welche zu einer verringerten Atmungskapazität und einer reduzierten olfaktorischen Wahrnehmung führt. Die molekularen Mechanismen, welche die Pathogenese und die Ausbildung von Nasenpolypen steuern, sind bislang nicht hinreichend verstanden. Die Anmelderin hat nunmehr den Einfluss des Monoterpenoxids 1,8-Cineol auf die Regulation des Wnt/β-Catenin-Signalwegs und die zelluläre Entwicklung von Nasenpolypen untersucht.

**Material und Methoden:** Die Anmelderin hat Gewebeproben aus Nasenpolypen und unteren Nasenmuscheln von Patienten mit CRSwNP (chronischer Rhinosinusitis mit Nasenpolypen) und aus unteren Nasenmuscheln von Probanden ohne chronische Sinusitis (gesunde Schleimhaut) auf Basis von immunohistochemischen Methoden und Immunoblot-Verfahren untersucht. Die zelluläre Proliferation und Migration wurden unter Einsatz des xCELLigence Real Time Cell Analyzer untersucht.

**Ergebnisse:** Im Rahmen dieser Arbeit konnte die Anmelderin erstmals zeigen, dass 1,8-Cineol als starker Inhibitor der Wnt/beta-Catenin-Aktivität wirkt und zu einem starken Rückgang der zellulären Entwicklung von Nasenpolypen führt. Die Daten der Anmelderin zeigen auf, dass 1,8-Cineol die inhibitorische Phosphorylierung von GSK-3-beta beeinflusst, welches der Schlüsselregulator der beta-Catenin-Aktivität ist.

**Fazit:** Die Ergebnisse der Anmelderin ermöglichen neue Erkenntnisse in das für die Ausbildung von Nasenpolypen verantwortliche regulatorische Netzwerk und zeigen darüber hinaus einen neuen Mechanismus der Aktivität von 1,8-Cineol, welcher einen neuen Behandlungsansatz dieses natürlichen Wirkstoffs darstellt.

### Einführung

Nasenpolypen sind krankhafte, jedoch gutartige ödematöse glasige Läsionen, welche aus der Nasenschleimhaut der Nasennebenhöhle und der Nasenhaupthöhle hervorgehen. Nasenpolypen sind eine verbreitete Krankheit mit einer allgemeinen Prävalenz von 1 bis 4 % in Europa, wobei zwischen chronischer Rhinosinusitis (CRS) mit Nasenpolypen (CRSwNP) und ohne Nasenpolypen (CRSsNP) unterschieden wird. Zahlreiche Studien gehen von einem Zusammenhang zwischen CRS und verschiedenen Faktoren, wie Luftverschmutzung, Tabakkonsum oder ganzjähriger allergischer Rhinitis, aus. Weiterhin spricht viel für eine Verbindung von CRS mit Asthma bronchiale, unabhängig von dem individuellen Rauchverhalten.

Das Monoterpenoxid 1,8-Cineol, bei welchem es sich um die Wirksubstanz des klinisch zugelassenen Medikaments (Soledum®) handelt, wird üblicherweise zur Behandlung verschiedener chronischer und akuter Erkrankungen der Atemwege sowie von Patienten mit chronischer Rhinosinusitis ohne Nasenpolypen angewendet. Obwohl ein positives Ansprechen dokumentiert wird, ist der molekulare Mechanismus des Einflusses auf die Entwicklung und Rezidive von Nasenpolypen immer noch nicht verstanden.

Im Rahmen der vorliegenden Studie hat die Anmelderin den Einfluss von 1,8-Cineol auf die Regulation des Wnt/beta-Catenin-Signalwegs mit den zentralen Regulatorproteinen Glykogen-Synthase-Kinase 3 (GSK-3) und beta-Catenin untersucht. Der Wnt/beta-Catenin-Signalweg ist an der Regulation verschiedener zellulärer Funktionen, wie Zelldifferenzierung, Proliferation, Migration und Adhäsion, beteiligt.

Zahlreiche Studien gehen von einer aktiven Rolle der Glykogen-Synthase-Kinase-3 (GSK-3) in verschiedenen Krankheiten, wie Alzheimer, Krebs, Entzündungen, kardiovaskulären Krankheiten oder Diabetes, aus. Derzeit sind zwei verschiedene Isoformen von GSK-3 bekannt, nämlich GSK-3-alpha und GSK-3-beta, welche durch zwei verschiedene Gene kodiert werden.

Im Gegensatz zu anderen Kinasen sind die Enzyme konstitutiv aktiv und werden in Antwort auf zelluläre Signale inaktiviert. Der APC/Axin/GSK-3-Komplex löst eine koordinierte Phosphorylierung von beta-Catenin aus, was zu dessen Ubiquitinierung und proteosomaler Degradation durch den beta-TrCP/SKP-Weg führt.

Durch die Anmelderin wurde nun gezeigt, dass die Aktivität von GSK-3 selbst durch verschiedene Phosphorylierungsmuster gesteuert wird. Während die Phosphorylierung von Serin-9 in GSK-3-alpha oder Serin-21 in GSK-3-beta zu einer verringerten Enzymaktivität führt, wird als Antwort auf eine Phosphorylierung der Reste Tyrosin-279 in GSK-3-alpha und Tyrosin-216 in GSK-3-beta eine stark ansteigende enzymatische Aktivität detektiert.

Im Rahmen der Studien der Anmelderin konnte die Anmelderin erstmals demonstrieren, dass 1,8-Cineol als starker Inhibitor der Wnt/beta-Catenin-Aktivität fungiert und zu einer stark verringerten zellulären Progression führt. Somit zeigen die Ergebnisse der Anmelderin einen neuen Mechanismus der Aktivität von 1,8-Cineol auf und führen folglich zu neuen Behandlungsansätzen für diesen natürlichen Wirkstoff.

### Material und Methoden

### Menschliche Gewebeproben

Die Anmelderin hat Gewebeproben aus Nasenpolypen und unteren Nasenmuscheln als interne Kontrolle mit CRSwNP untersucht, welche im Rahmen von chirurgischen Eingriffen an den Nasennebenhöhlen entnommen wurden.

Alle Patienten hatten in der Vergangenheit über einen Zeitraum von mindestens 3 Monaten mit einer mittleren Dauer von 1,5 Jahren (+/-0,6 Jahren) Beschwerden an den Nebenhöhlen, wobei konservative Therapien fehlgeschlagen waren. Vor den chirurgischen Eingriffen wurden die Patienten im Rahmen von Unverträglichkeits-Hauttests unter Einsatz von standardisierten Extrakten (Allergopharma Joachim Ganzer KG, Reinbek, Deutschland) auf Pollen, Hausstaubmilbe, Haustiere und Schimmel getestet. Durch histopathologische Untersuchung wurde die eosinophile CRSwNP bestimmt; kein Patient mit neutrophilen Polypen oder insbesondere mit Mukoviszidose wurde in die Studie aufgenommen. Keiner der Patienten war in einem Zeitraum von 4 Wochen vor dem chirurgischen Eingriff mit systemischen oder topischen Kortikosteroiden behandelt worden. Darüber hinaus wurden Proben der unteren Nasenmuscheln von Patienten ohne Sinusitis oder Allergien in der Vergangenheit, welche sich chirurgischen Eingriffen am Septum oder einer Septorhinoplastik unterzogen, als interne Kontrolle entnommen. Die Studie wurde durch die medizinische Ethikkommission der Universität Lübeck genehmigt, nach den Weisungen der ethischen Vorschriften für medizinische Forschung, wie sie in der WMA Deklaration von Helsinki formuliert sind, ausgeführt und alle Patienten haben eine unterzeichnete Einverständniserklärung abgegeben.

### 1,8-Cineol

Es wurde 1,8-Cineol aus Soledum®-Kapseln (Klosterfrau Healthcare Group, Cassella-med GmbH & Co. KG, Köln, Deutschland) eingesetzt.

Die aus Eukalyptusöl isolierte Reinsubstanz, d. h. reines 1,8-Cineol mit einer Reinheit > 99 % (0,6 mg/µl bzw. 600 mg/ml 1,8-Cineol) wurde bei 4 °C gelagert, wohingegen die Stammlösung durch Lösen der Reinsubstanz in Ethanol (100 mg/ml), gefolgt von einer endgültigen Verdünnung mit "DMEM High Glucose" (Biochrom, Berlin, Deutschland; 1 mg/ml), hergestellt wurde.

### Herstellung von Einzelzellsuspensionen

Proben von Nasenpolypen wurden in PBS-Puffer gewaschen und in sterilem serumfreiem "High Glucose Dulbecco's Modified Eagle Medium" (PAA, Pasching, Österreich) mit einer Skalpellklinge vorsichtig in kleine Stücke zerkleinert, wobei das Medium 1 % L-Glutamin, 1 % Natriumpyruvat, 1 % nichtessentielle Aminosäuren, 2 % Amphotericin B, 2 % Streptomycin und 2 % Penicillin, ergänzt mit 10 % FCS, und 25 mM HEPES Puffer, enthielt (alle von PAA, Pasching, Österreich). Danach wurden die Gewebeproben durch eine enzymatische Behandlung und Waschschritte mit Collagenase Typ II (31,5 mg/ml, GIBCO, Eggenstein, Deutschland) und Hyaluronidase (3,99 mg/ml, Sigma, München, Deutschland) in einem ersten Schritt und mit Dispase (33,4 mg/ml, Sigma, München, Deutschland) in einem zweiten Schritt jeweils unter Schütteln bei 37 °C für 120 Minuten enzymatisch verdaut. Zwischen den enzymatischen Behandlungen und den Waschschritten wurden die Zellen zentrifugiert. Die resultierenden Zellsuspensionen wurden in PBS (PAA, Pasching, Österreich), enthaltend 2 % BSA, gewaschen und jeweils durch einen 70 µm und einen 60 µm Nylon-Zellfilter (Falcon, Becton Dickinson Labware, Heidelberg, Deutschland) filtriert.

### MTT-Test

Die Zellproliferation wurde mittels eines quantitativen kolorimetrischen 3-(4,5-Dimethylthiazol 2-yl)-2,5-diphenyltetrazoliumbromid-basierten (MTT) Zelltests bestimmt. Dieser Test bestimmt die Anzahl an lebensfähigen Zellen auf Basis der mitochondrialen Umwandlung von MTT.

In jedes Well einer 96-Well-Platte wurden jeweils 5.000 Zellen verteilt. Nach 24 Stunden Kultivierung wurden vier verschiedene Konzentrationen von 5-Fluorouracil oder Cisplatin den Kulturen zugefügt oder die Zellen wurden mit drei Dosen bestrahlt. Nach 48 Stunden wurden 10 µl des MTT-Farbstoffs (5 mg/ml) jedem Well zugefügt. Nach zwei Stunden Inkubation mit MTT wurden die Kristalle gelöst und für 24 Stunden bei Raumtemperatur vorsichtig geschüttelt. Unter Einsatz eines Mehrfach-Well-ELISA-Lesegeräts wurde die Absorption der reduzierten Formazan-Produkte in Kontrolle und Versuchswells bei einer Wellenlänge von 570 nm und 690 nm ausgelesen. Die MTT-Tests wurden jeweils dreifach durchgeführt.

### Analyse von Immunfärbungen

Frische eingefrorene Gewebeschnitte wurden für 10 Minuten in 100 % Methanol fixiert. Nach einer Trocknungszeit von 10 Minuten wurden die Schnitte bei Raumtemperatur mit TBS (RT-TBS) gewaschen. Danach wurden die Gewebeproben zur Inhibition der endogenen Peroxidase 15 Minuten lang in RT-TBS mit 3 % H₂O₂ inkubiert.

Nach einem Waschschritt mit RT-TBS wurden die Proben in TBS mit 0,3 % Triton X für 15 Minuten permeabilisiert. Die Schnitte wurden in einer 1 : 100-Verdünnung mit Anti-Phospho-GSK-3-alpha/beta-Antikörpern (Cell Signaling Technology Inc., Massachusetts, USA, Katalognummern 5676 und 9331) über Nacht bei 4 °C inkubiert.

Am nächsten Tag wurden die Objektträger mit RT-TBS gewaschen und danach für 20 Minuten bei Raumtemperatur mit einem sekundären Polylink-Antikörper inkubiert. Nach dem Waschen mit RT-TBS wurden die Proben mit Peroxidase-Marker (HRP) (beide DCS Detection line, Innovative Diagnostik-Systeme, Hamburg, Deutschland) inkubiert.

Nach dem Waschen mit RT-PBS wurden die Schnitte in AEC (DCS Chromoline, Innovative Diagnostik-Systeme, Hamburg, Deutschland) inkubiert. Die Reaktion wurde mit RT-TBS gestoppt. Schließlich wurde eine Zellkernfärbung mit Mayer's Haematoxylin durchgeführt. Die Schnitte wurden in wässrigem "Dako Faramount Medium" (Dako North America Inc., Carpinteria, CA, USA) eingebettet. Die immunogefärbten Schnitte wurden mit einem konfokalen Mikroskop (Axiovert 200, Carl Zeiss, Oberkochen, Deutschland) betrachtet und mikrofotografisch aufgenommen.

### Herstellung von Zell-Lysaten

Proben von Nasenpolypen wurden in PBS gewaschen und vorsichtig homogenisiert. Es wurden Zellextrakte hergestellt und unter Verwendung von Lysepuffer 16 ("Human pluripotent stem cell array" (R&D Systems)) solubilisiert. Die Proteinkonzentrationen wurden bestimmt.

### Zellproliferations-Assay

Die Proliferation wurde mittels eines MTT-Tests von Sigma-Aldrich ("In Vitro Toxicology Assay Kit") entsprechend den Instruktionen des Herstellers ermittelt.

### Western Blot

Lysate von Geweben wurden durch Erhitzen über einen Zeitraum von 5 Minuten in einfachem SDS-Probenpuffer (Carl Roth GmbH, Karlsruhe, Deutschland) denaturiert. Die Proteinkonzentrationen wurden mittels des Bradford-Tests bestimmt und jeweils 30 µg Gesamtprotein wurden zur SDS-Polyacrylamid-Gel Elektrophorese eingesetzt (Mini-Protean TGX®, Any kD, BioRad, Hercules, CA, USA) und anschließend auf Nitrozellulosemembranen übertragen. Die Übertragung der Proteine wurde mittels Ponceau-Färbung kontrolliert.

Zunächst wurden die Membranen mit TBS, welcher 0,05 % TWEEN 20 (TBS-T) und 5 % BSA (BioRad, Hercules, CA, USA) enthielt, über eine Dauer von 1 Stunde bei Raumtemperatur geblockt und anschließend über Nacht bei 4 °C in einer Verdünnung von 1 : 1.000 mit dem jeweiligen primären Antikörper in PBS-T und 5 % BSA inkubiert: Anti-GSK-3-alpha/beta (Abcam plc., Cambridge, Vereinigtes Königreich, Katalognummer ab62368) und Anti-Phospho-GSK-3-alpha/beta (Serin-9/Serin-21), (Cell Signaling Technology Inc., Massachusetts, Katalognummer 9331). Nach einem Waschschritt mit PBS-T wurden die Membranen mit einem Ziege-anti-Kaninchen-spezifischen sekundären Antikörper (BioRad, Hercules, CA, USA), welcher in einer Verdünnung von 1 : 3333 vorlag, in TBS-T mit 5 % BSA über eine Dauer von einer Stunde bei Raumtemperatur inkubiert. Die Proteinbanden wurden mit Hilfe eines Konjugat-Substrat-Kit auf Basis von alkalischer Phosphatase (BioRad, Hercules, CA, USA) visualisiert.

Um die Beladungsmenge jeder Spur zu bestimmen, wurden die Nitrozellulosemembranen über Nacht bei 4 °C mit einem primären Antikörper der GAPDH (Cell Signaling Technology Inc., Massachusetts, Katalognummer: 14C10) in einer Verdünnung von 1 : 1.000 inkubiert. Die Membranen wurden gewaschen und danach mit einer 1 : 1.000-Verdünnung eines Speziesspezifischen Meerrettich-Peroxidase (HRP) gekoppelten sekundären Antikörpers in PBS-T und 5 % BSA für drei Stunden bei Raumtemperatur inkubiert. Die Membranen wurden gewaschen und die Proteinbanden wurden wie zuvor beschrieben visualisiert. Die Pixeldichten wurden quantifiziert und mittels Fusion FX7® unter Einsatz von Bio-1D Software® dokumentiert.

### Zellmigrations-Test durch xCELLigence

Um den Einfluss von Cineol auf die zelluläre Migrations-Aktivität zu untersuchen, wurden Migrations-CIM-Platten 16 (Roche, Mannheim, Deutschland), bei welchen es sich um ein durch eine poröse Membran getrenntes 2-Kammer-System handelt, entsprechend der Bedienungsanleitung des Herstellers verwendet (Roche Diagnostics). Die Zellen wandern dabei von der oberen Kammer durch die Membran und haften an den elektronischen Sensoren an der Unterseite der Membran, so dass ein Anstieg der Impedanz resultiert. Die Veränderungen werden automatisch und kontinuierlich durch das RTCA-DP-Instrument aufgezeichnet, und die Zellmigrations-Aktivität kann durch das Zell-Index-Profil überwacht werden. Vor dem Befüllen der Wells mit Zellen (60.000 Zellen/Well) wurde die Unterseite der Wells der oberen Kammer der CIM-Platte 16 mit 30 µl Collagen I (400 mg/ml, Sigma, Deisenhofen, Deutschland) beschichtet. Jede Behandlungsbedingung wurde in dreifacher Ausführung gemessen.

### Ergebnisse

### Cineol führt zu einer stark verringerten Entwicklung von Nasenpolypen

Im Vergleich zur gesunden Nasenschleimhaut weisen Nasenpolypen eine andere Morphologie auf, was anhand von immunohistochemischen Färbungen mit Hämatoxylin-Eosin (HE) an gefrorenen Gewebeschnitten gezeigt werden konnte (Figur 1). Mit dieser Färbemethode werden basophile Strukturen mit Blau-Lila und eosinophile Strukturen mit hellem Pink angefärbt, wohingegen hydrophobe Strukturen, wie die Membranen des Golgi-Apparats, ungefärbt bzw. klar bleiben. Die Anmelderin hat den Einfluss von 100 µM 1,8-Cineol auf die zelluläre Proliferations-Aktivität von Nasenpolypen untersucht, wobei der xCELLigence Real Time Cell Analyzer eingesetzt wurde. Als Reaktion auf eine Inkubation in 100 µM 1,8-Cineol konnte nach 144 Stunden eine stark verringerte Proliferation von Nasenpolypenzellen beobachtet werden (vgl. Figur 2A, mit zwei verschiedenen Probanden I und II). Überraschenderweise wurden kultivierte Zellen aus der Nasenschleimhaut durch die Behandlung mit 1,8-Cineol nicht beeinflusst, was eine kontextspezifische Wirkweise von 1,8-Cineol nahelegt (vgl. Figur 2B).

### Verringerte beta-Catenin-Aktivität als Antwort auf die Behandlung mit 1,8-Cineol

Um den Einfluss von 1,8-Cineol auf die Aktivität und Regulation des Wnt/beta-Catenin-Signalwegs zu untersuchen, hat die Anmelderin zunächst das Expressionslevel von aktivem beta-Catenin analysiert, bei welchem es sich um das zentrale transkriptionelle Aktivator-Protein dieses Signalweges handelt. Die Daten der Anmelderin zeigen einen starken Rückgang von aktivem beta-Catenin als Reaktion auf die Behandlung mit 1,8-Cineol in Nasenpolypen, verglichen mit den Medium-Kontrollen. Als Reaktion auf 24 Stunden Inkubation mit 100 µM 1,8-Cineol verschwanden aktive beta-Catenin-Proteine fast vollständig in den analysierten Gewebeproben von Nasenpolypen. Diese Daten zeigen eine starke Inhibition des Wnt/betaß-Catenin-Signalweges durch die Behandlung mit 1,8-Cineol auf (Figur 3).

### GSK-3-Aktivität als Antwort auf die Behandlung mit 1,8-Cineol

In einem nächsten Schritt wurden zusätzliche Untersuchungen durchgeführt, um die korrespondierenden Phosphorylierungs-Level des beta-Catenin-Regulators GSK-3 zu analysieren. Bei GSK-3 handelt es sich um ein konstitutiv aktives Protein, welches durch Phosphorylierung von GSK-3 an Serin-9 (GSK-3-alpha) oder Serin-21 (GSK-3-beta) inhibiert wird. Um die Expression und Phosphorylierungs-Level von GSK-3-alpha/beta in Anwesenheit und Abwesenheit von 100 µM 1,8-Cineol zu untersuchen, wurden Western Blot-Experimente durchgeführt.

Die Daten der Anmelderin zeigen deutlich, dass Cineol zu einer verringerten Phosphorylierung von GSK-3-alpha/beta an Serin-9/-21 führt, was wiederum zu einer verstärkten Aktivität von GSK-3 und somit zu einer verstärkten Degradation von beta-Catenin führt (Figur 4A). Neben der Phosphorylierung an Serin-9/-21 wird die GSK-3-Aktivität auch auf dem Level seiner subzellulären Lokalisierung reguliert. GSK-3 kann zur Trennung von seinen Substraten in endolysosomale Vesikel verlagert werden, um die Kinase-Aktivität zu verringern. Die immunohistochemischen Untersuchungen der Anmelderin zeigen eine verringerte endolysosomale Lokalisierung von GSK-3 als Reaktion auf 100 µM 1,8-Cineol, was dessen verringerte Inhibition nach der Behandlung mit 1,8-Cineol hervorhebt (Figur 4B). GSK-3 ist in die Regulation von zahlreichen biosynthetischen Signalwegen involviert und zahlreiche Kinasen sind in der Lage, die GSK-3-Aktivität durch Phosphorylierung an dessen aktivierenden oder hemmenden Phosphorylierungsstellen zu regulieren. Die Akt-Kinase (auch bekannt als PKB) wurde als Onkogen mit Serin/Threonin-Kinase-Aktivität identifiziert und es wurde gezeigt, dass diese *in vivo* einen negativen Regulator der GSK3-beta Aktivität darstellt. Akt kann durch Phosphorylierung an Serin-473 durch PDK1 aktiviert werden, wobei davon ausgegangen wird, dass PHLPP als Inhibitor von dessen Phosphorylierung fungiert. In einem ersten Ansatz hat die Anmelderin die Expressionslevel und Serin-473-Phosphorylierungslevel von Akt in Reaktion auf 100 µM 1,8-Cineol analysiert. Die Phosphorylierungslevel von Akt an Serin-473 wurden in allen analysierten Proteinextrakten auf Basis von Nasenpolypen nicht wesentlich beeinflusst, wenn auch individuelle Expressionslevel gemessen wurden (Figur 5).

### Diskussion

Bei Nasenpolypen handelt es sich um eine benigne, proliferative Krankheit der Nasenschleimhaut und der mittleren Nasenmuscheln. Die gegenwärtige gemäß internationalen und europäischen Leitlinien empfohlene Standardtherapie basiert auf topischen Kortikosteroiden. Das Ansprechen von Nasenpolypen auf Steroide ist jedoch oftmals nicht zufriedenstellend, selbst wenn diese als Langzeittherapie mit zwischenzeitlichen Zyklen von systemischen Glucokortikoiden appliziert werden, so dass oft dennoch chirurgische Maßnahmen notwendig sind.

Die neuesten Daten der Anmelderin zeigen in Nasenpolypen im Vergleich zu den unteren Nasenmuscheln von Patienten mit CRSwNP eine signifikant höhere Phosphorylierungsrate von GSK-3-alpha/beta. Weiterhin hat die Anmelderin eine gleichmäßige Expression von pGSK-3-alpha/beta in allen Zellen der Epithelschicht in Polypen mit Verdickungen des Epithels beobachtet.

Bei GSK-3 handelt es sich um ein vielseitiges Enzym, welches in eine Vielzahl von zellulären Signalkaskaden involviert ist. GSK-3 ist zahlreichen Hauptsignalwegen nachgeschaltet, umfassend den Phosphatidylinositol 3'-Kinase-Signalweg, den Wnt-Signalweg, die Hedgehog-Signalgebung und den Notch-Signalweg.

Zahlreiche Reize führen zu einer Inaktivierung von GSK-3, umfassend Wachstumsfaktoren wie EGF (Epidermal Growth Factor), PDGF (Platelet-Derived Growth Factor), BDNF (Brain-derived neurotrophic factor), IGF (Insulin-like growth factor) und Insulin.

Interaktionen von GSK-3 mit NF-kB, mTOR, TGF-beta und p38 sind bekannt. GSK-3 phosphoryliert eine Vielfalt an Substraten, z. B. die Glykogen-Synthase und andere Stoffwechselenzyme, β-Catenin, die Transkriptionsfaktoren CBP (CREB Binding Protein), c-Myc und c-Jun und die Translationsinitiationsfaktoren eIF2 und eIF2B.

In vorherigen Untersuchungen hat die Anmelderin eine Aktivierung von GSK-3 in entzündeter Nasenschleimhaut im Allgemeinen und anderweitig eine Aktivierung von GSK-3 in Nasenpolypen beobachtet.

Im Rahmen dieser Arbeit hat die Anmelderin nun erstmals gezeigt, dass 1,8-Cineol als starker Inhibitor der Wnt/beta-Catenin-Aktivität fungiert und dabei zu einem starken Rückgang der zellulären Progression führt.

Die verringerte Degradation von beta-Catenin korreliert mit einer verringerten Inhibition von GSK3-beta, wobei die für diesen Mechanismus verantwortlichen angesprochenen Proteine nicht weiter aufgeklärt worden sind (vgl. Figur 6 als Modell).

Zusammenfassend lässt sich feststellen, dass die Ergebnisse der Anmelderin einen neuen Therapieansatz bzw. Wirkeffekt für die 1,8-Cineol-Aktivität aufzeigen und zu neuen Behandlungsanwendungen dieses natürlichen bzw. naturbasierten Wirkstoffs führen.

### Beschreibung der Figuren

### Figur 1

### Hämatoxylin-Eosin (HE)-Färbung von Nasenpolypen und gesunder Nasenschleimhaut

Diese Färbemethode färbt basophile Strukturen blau-lila und eosinophile Strukturen in hellem Pink, wohingegen hydrophobe Strukturen, wie die Membranen des Golgi-Apparats, ungefärbt bzw. klar bleiben.

### Figur 2

### Dosisabhängige Reduktion der zellulären Progression durch 1,8-Cineol

Um zu bestimmen, ob die Behandlung mit 1,8-Cineol zu einer Inhibierung der zellulären Lebensfähigkeit von Nasenpolypen führt, wurden MTT-Tests eingesetzt. Eine stark abnehmende Proliferation von Nasenpolypen-Zellen konnte als Reaktion auf eine Inkubation mit 100 µM 1,8-Cineol nach 144 Stunden beobachtet werden (Figur 2A, mit zwei verschiedenen Probanden I und II). Überraschenderweise wurden kultivierte Epithelzellen der Nasenschleimhaut durch Behandlung mit 1,8-Cineol nicht beeinflusst, was einen kontextabhängigen spezifischen Wirkmechanismus von 1,8-Cineol nahelegt (Figur 2B).

### Figur 3

### Expression von aktivem beta-Catenin auf Antwort auf Cineol

Um den Effekt einer 24 Stunden-Behandlung mit 100 µM 1,8-Cineol auf das Protein-Expressions-Level von aktivem beta-Catenin in drei verschiedenen Gewebeproben von Nasenpolypen (A, B, C) zu untersuchen, wurden Western-Blot-Analysen an Gesamtproteinen durchgeführt. GAPDH wurde als Beladungskontrolle eingesetzt.

### Figur 4

### (A) Expression von aktivem beta-Catenin und P-GSK-3 (Serin-9/-21) als Reaktion auf Cineol

Um den Effekt einer 24-stündigen Behandlung mit 100 µM 1,8-Cineol auf die Protein-Expression-Level von aktivem beta-Catenin, Phospho-GSK-3 (Serin-9/-21) und Phospho-GSK-3 (Tyrosin-279/-216) in vier verschiedenen Proben von Nasenpolypen (D, E, F, G) zu untersuchen, wurden Western-Blot-Analysen an Gesamtproteinen durchgeführt. GAPDH wurde als Beladungskontrolle verwendet.

### (B)/(C) Subzelluläre Lokalisation von P-GSK-3 (Serin-9/-21) als Reaktion auf Cineol

Um eine verringerte endolysosomale Lokalisierung von Phospho-GSK-3 (Serin-9/-21) als Reaktion auf 100 µM 1,8-Cineol, verglichen zum Kontrollmedium, zu untersuchen, wurden immunohistochemische Untersuchungen durchgeführt. Zellkerne wurden mit DAPI angefärbt.

### Figur 5

### Expression von Akt und P-Akt (Serin-473) als Reaktion auf 1,8-Cineol

Western Blot-Analysen an Gesamtproteinen wurden eingesetzt, um den Effekt von 100 µM 1,8-Cineol auf die Expressions-Level von Akt und P-Akt (Threonin-308) in vier verschiedene Gewebeproben von Nasenpolypen zu zeigen (H, I, J, K). GAPDH wurde als Beladungskontrolle verwendet.

### Figur 6

### Modell der Wnt/beta-Catenin-Inhibition als Reaktion auf 1,8-Cineol

Im Rahmen dieser Arbeit hat die Anmelderin erstmals gezeigt, dass 1,8-Cineol als Inhibitor für die Wnt/beta-Catenin-Aktivität fungiert und einen starken Rückgang der zellulären Progression von Nasenpolypen bewirkt. Die Daten der Anmelderin zeigen, dass 1,8-Cineol die inhibitorische Phosphorylierung und endolysosomale Lokalisierung von GSK3-beta, bei welchem es sich um den zentralen Regulator der beta-Catenin-Aktivität handelt, beeinflusst.

### 3. Klinische Anwendungstests betreffend die Verwendung von 1,8-Cineol bei Patienten mit Polyposis nasi et sinuum

Im Rahmen einer klinischen Anwendung erhielten 22 Patienten nach einer Nasennebenhöhlenoperation zusätzlich zu einer Standardmedikation 1,8-Cineol (Soledum forte®), 3-mal 1 Kapsel, über einen Zeitraum von 3 Monaten. Die präoperativen CT-Bilder wurden nach dem Lund-Mackay-Score bewertet. Die Patienten wurden gebeten, ihre Beschwerden mittels eines Fragebogens (SNOT20 - *German adapted version*) einzuschätzen. 21 von 22 Patienten beendeten die Therapie wie vorgesehen. Ein Patient berichtete über gastrointestinale Nebenwirkungen und brach die Behandlung mit 1,8-Cineol ab. Die Nachuntersuchung nach 6 Monaten zeigte lediglich bei einem Patienten eine beginnende geringfügige Rezidivpolyposis. Bei einer ersten orientierenden Auswertung war eine deutliche Verbesserung des SNOT-20-Scores im Vergleich zu den präoperativen Ergebnissen zu beobachten. Somit war ein gutes Ansprechen auf die zusätzliche medikamentöse Behandlung mit 1,8-Cineol bei einem tolerablen Nebenwirkungsprofil zu beobachten.

1,8-Cineol ist somit eine wertvolle Ergänzung der medikamentösen Nachbehandlung nach Nasennebenhöhlenchirurgie.

Die bisherigen vielversprechenden klinischen Anwendungstests zu den von der Anmelderin neu aufgefundenen Wirkeffekten und Therapiemöglichkeiten von 1,8-Cineol werden fortgeführt und alle bislang mit 1,8-Cineol behandelten Patienten werden regelmäßig hinsichtlich der Entstehung möglicher Rezidive untersucht.

Darüber hinaus sollen nun die Wirkeffekte von 1,8-Cineol auch bei adenoider Konstitution untersucht werden und in diesem Zusammenhang steht auch die Evaluation der möglichen Verfügbarkeit von 1,8-Cineol in Form eines Nasensprays.

### 4. Weitergehende Studien: Klinische und molekularbiologische Ansätze zum therapeutischen Einsatz von Cineol, insbesondere 1,8-Cineol, bei Nasenpolypen (Polyposis nasi et sinuum)

### 1. Hintergrund

*Polyposis nasi et sinuum* sind Ausstülpungen von chronisch entzündeter und ödematöser Schleimhaut aus Siebbeinzellen, Keilbeinhöhle und Kieferhöhle in die Nasenhöhle. Sie können zu Behinderung der Nasenatmung, Geruchsverlust und weiteren Erkrankungen der Nasennebenhöhlen führen. Gering ausgeprägte Erkrankungen können durch die Einnahme von Glukokortikoiden (kortisonhaltige Nasensprays, Kortisontabletten etc.) behandelt werden. Zumeist ist jedoch eine chirurgische Therapie, um die Nasenatmungsbehinderung und den Geruchsverlust wirksam zu behandeln. Die Operationen werden endonasal (durch die Nasenhöhlen) und in Allgemeinnarkose minimal invasiv durchgeführt und erreichen Heilungsraten von etwa 50 % und eine Beschwerdebesserung in 90 % der Fälle. *Polyposis nasi et sinuum* und chronisch-polypöse Nebenhöhlenerkrankungen neigen zu Rezidiven. Oft sind im Krankheitsverlauf mehrere Operationen erforderlich. Minimal-invasive Nebenhöhlenoperationen zählen zu den anspruchsvollen Eingriffen im HNO-Bereich und sind für Patienten vielfach mit einem mehrtägigen Krankenhausaufenthalt und intensiver Nachbehandlung verbunden.

Im strengen Sinne versteht man unter einer Rhinitis die entzündliche Veränderung der Nasenschleimhaut und unter einer Sinusitis gleichartige Veränderungen der Mucosa der Nasennebenhöhlen. Im Zentrum der Beschwerden stehen Schmerzen, eine Rhinorrhö und die Behinderung der Nasenatmung. Epidemiologie, kausale und formale Pathogenese der Rhinosinusitis sind nur in wenigen Fällen befriedigend geklärt. Eine Klassifikation der Rhinitis und/oder Sinusitis kann nach der Anamnese (Frequenz und Dauer der Beschwerden), nach immunologischen und morphologischen Gesichtspunkten oder versuchsweise nach ihrer Ätiologie oder Assoziation zu systemischen Erkrankungen vorgenommen werden.

Bei den häufigsten Entitäten der Rhinitis wurde ein fließender Übergang von Rhinitis und Sinusitis festgestellt. Die Schleimhaut von Nase und Nasennebenhöhlen unterscheidet sich in Struktur und Funktion weniger qualitativ als vorwiegend quantitativ, sie geht im Bereich der Nebenhöhlen-Ostien direkt ineinander über. In Übereinstimmung mit diesem Bild führt selbst ein banaler viraler Schnupfen in der überwiegenden Anzahl von Fällen (bis zu 87 %) zu einer im Computertomogramm nachweisbaren Mitbeteiligung der Nasennebenhöhlen. Es werden anhand von Anamnese und Befunden 3 bis 5 Typen der Rhinosinusitis unterschieden.

Bei der chronischen Rhinosinusitis stellt die Schleimhautschwellung mit Verlegung der Nebenhöhlen-Drainagewege nur einen der pathogenetischen Faktoren dar, in einem anderen Teil der Fälle kommt es im Zusammenwirken weiterer, oft noch weitgehend unbekannter Faktoren, wie z. B. zur Ausbildung von Nasenpolypen als gutartige, wässrige, oft grau-glasige Schleimhauthyperplasien. Aus diesem Grund wird in der Literatur eine Trennung zwischen polypöser und nicht polypöser Rhinosinusitis vorgenommen. Die vorliegende Anmeldung fokussiert auf die chronisch polypöse Sinusitis möglichst mit Ausprägung einer *Polyposis nasi et sinuum.*

### Konservative Therapie:

### Glukokortikoide lokal bei Polyposis nasi et sinuum

Insbesondere die Eosinophilen-assoziierte Entzündungsreaktion in Nasenpolypen wird von Glukokortikoiden effektiv gehemmt. Hierbei kommt es klinisch zu einer deutlichen Reduktion der Symptomatik. In den letzten Jahren wurden die klinischen Beobachtungen zunehmend durch objektive Parameter gestützt (Rhinomanometrie, Rhinometrie, Peak nasal inspiratory flow PNIF, Magnetresonanz-Tomographie). Auch die Rezidivrate bzw. der Zeitpunkt des Rezidivs nach Operation wurden in einigen Studien signifikant verbessert bzw. hinausgezögert, die Untersuchungen gehen allerdings über den Zeitraum eines Jahres nicht hinaus. Der Einsatz von topischen Kortikosteroiden über mehrere Monate bei unbehandelten Nasenpolypen sowie als Beginn eines Therapie-Schemas vor einer chirurgischen Sanierung sowie zur Rezidivprophylaxe nach einer chirurgischen Therapie (6 Monate bis 1 Jahr) ist damit zu empfehlen.

### Chirurgische Therapie

### Indikationen zu Nasennebenhöhleneingriffen

Die operative Intervention bei der rezidivierenden und chronischen Rhinosinusitis mit oder ohne *Polyposis nasi et sinuum* ist dann indiziert, wenn die konservative Therapie keine oder keine dauerhafte Besserung der Beschwerden bringt.

### Zusammenfassung:

Es gibt in der Literatur bislang keine Untersuchungen, die den Einfluss von Cineol oder ähnlichen Substanzen in der Therapie der chronisch polypösen Sinusitis (mit oder ohne *Polyposis nasi et sinuum*) untersucht. Bislang ist hier nur der Einsatz von oralen und/oder topischen Glukokortikoiden untersucht und standardisiert. Ziel ist es zu untersuchen, inwieweit sich ähnliche Ergebnisse in der Behandlung der chronisch polypösen Sinusitis (mit oder ohne *Polyposis nasi et sinuum*) nachweisen lassen. Wie bereits vorstehend dargelegt, ist die Anmelderin von zwei Seiten vorgegangen, nämlich einerseits von der klinischen Seite sowie andererseits von der molekularbiologischen Seite.

### 2. Vorarbeiten zum Forschungsprojekt

### Stammzellcharakteristika von Polyposis nasi et sinuum

Nasenpolypen zeigen, verglichen mit gesunder Nasenschleimhaut, eine stark abweichende Morphologie, welche in Figur 1 durch eine Hematoxylin-Eosin (HE) Färbung von Gefrierschnitten beider Gewebe verdeutlicht wird (Figur 1). Diese Färbemethode färbt basophile Strukturen in Blau-purpur und eosinophile Strukturen in Pink an, während hydrophobe Strukturen wie etwa die Membranen des Golgi Apparates klar bleiben. Figur 1 zeigt somit die unterschiedliche Morphologie von gesunder Nasenschleimhaut und *Polyposis nasi et sinuum.*

Um die Expressionsprofile unterschiedlicher Stammzellmarker in *Polyposis nasi et sinuum* zu untersuchen, wurde ein Protein-Array verwendet, der die parallele Analyse eines breiten Spektrums unterschiedlicher Stammzellproteine ermöglicht. Es wurden die Expressionslevel von 10 verschiedenen Stammzellmarkern ausgewertet, welche auf diese Weise in *Polyposis nasi et sinuum* identifiziert werden konnten. Zu diesen Proteinen gehören unter anderem die Transkriptionsaktivatoren SOX2, Nanog, SOX17, Pdx1 und Otx2, welche eine wichtige Rolle bei der Regulation zellulärer Differenzierungsprozesse spielen. Des Weiteren konnten die Proteine Tumor-Protein 63 (TP63), Alpha-Fetoprotein (AFP), Beta human chorionic gonadotropin (HCG) und E-Cadherin identifiziert werden, welche Schlüsselproteine unterschiedlicher zellulärer Prozesse wie Differenzierung, Proliferation und Adhäsion in humanen pluripotenten Stammzellen darstellen.

### Oct-4 A exprimierende Stammzellen in Polyposis nasi et sinuum

Unter den identifizierten Stammzellmarkern befindet sich auch der Transkriptionsaktivator Oct-4 in *Polyposis nasi et sinuum.* Es wird vermutet, dass Oct-4 der zentrale Regulator der embryonalen Pluripotenz und Selbsterneuerungskapazität ist.

Es wurde gezeigt, dass 2 unterschiedliche Isoformen von Oct-4, nämlich Oct-4 A und Oct-4 B existieren, welche sich sowohl in Proteingröße als auch in Funktion unterscheiden.

Es wird vermutet, dass lediglich die größere Isoform Oct-4 A Stammzelleigenschaften reguliert, während die Funktion von Oct-4 B weitestgehend unaufgeklärt ist.

Daher hat die Anmelderin Oct-4 A spezifische Antikörper verwendet, um die Existenz Oct-4 A exprimierender Zellen in *Polyposis nasi et sinuum* immunhistochemisch zu analysieren.

Die Daten zeigen deutlich das Vorhandensein Oct-4 A exprimierender Zellen in *Polyposis nasi et sinuum* und weiterführende durchflusszytometrische Untersuchungen zeigen durchschnittlich etwa 1,7 % Oct-4 A exprimierender Zellen in *Polyposis nasi et sinuum,* welche negativ für die analysierten Lineage-Marker CD3, CD16, CD19, CD20 und CD56 sind. Immunhistochemische und durchflusszytometrische Untersuchungen zeigen durchschnittlich etwa 1,7 % Oct-4 A exprimierender Zellen in *Polyposis nasi et sinuum*, welche negativ für die analysierten Lineage-Marker CD3, CD16, CD19, CD20 und CD56 sind.

### Inhibierender Einfluss von Cineol auf die Progression von Polyposis nasi et sinuum

Erste Vorversuche zum Einfluss von Cineol auf die Progression von *Polyposis nasi et sinuum* wurden durchgeführt. Hierzu wurden zunächst Einzelzellsuspensionen hergestellt und diese wurden in der Zellkultur über einen Zeitraum von 288 Stunden mit Standardmedium und mit 1mM Cineol inkubiert. Die Ergebnisse zeigen den massiven Abfall der Zellvitalität nach 96 bis 144 Stunden durch Cineol. Dies belegt einen inhibierender Einfluss von 1mM Cineol auf die Progression von *Polyposis nasi et sinuum.*

Diese Versuche wurden bislang mit Polypen von 5 unterschiedlichen Patienten durchgeführt und zeigten jedes Mal erstaunlich übereinstimmende Ergebnisse, so dass die Anmelderin die weiterführende Analyse der zugrunde liegenden molekularen Mechanismen der Wirkweise von Cineol, insbesondere auch auf die Stammzellcharakteristika und somit auf die Rezidivraten von *Polyposis nasi et sinuum,* für äußerst vielversprechend und therapierelevant erachtet.

### 3. Zielsetzung

Es soll der Einfluss von Cineol auf die molekularen Mechanismen und Signaltransduktionskaskaden bei der Progression von Polyposis nasi et sinuum, insbesondere auch im Hinblick auf die Stammzellcharakteristika dieser Erkrankung, umfassend charakterisiert werden.

### (I) Experimenteller Teil

### Einfluss von Cineol auf Zellvitalität und Proliferation

Es wurden weiterführende Untersuchungen durchgeführt zur Analyse des Einflusses von Cineol auf die Vitalität und Proliferation verschiedener permanenter Zelllinien. Hierzu wurden einerseits die zwei HNSCC-Zelllinien und des Weiteren eine Fibroblastenzelllinie verwendet. Diese wurden über einen Zeitraum von 250 Stunden mit verschiedenen Cineol-Konzentrationen kultiviert (1 mM bis 10 mM) und die Vitalität der Zellen wurde jeweils mittels eines MTT-Assays untersucht. Es wurde sowohl ein inhibierender Einfluss von Cineol auf die Progression und Vitalität von HNSCC als auch ein inhibierender Einfluss von Cineol auf die Progression und Vitalität von Fibroblasten gefunden.

Hierbei zeigte sich ein ähnliches Bild wie in den vorhergegangenen Untersuchungen zum Einfluss von Cineol auf die Vitalität von *Polyposis nasi et sinuum*-Einzelzellsupensionen, wobei sich jedoch die permanenten Zelllinien erst zu geringfügig späteren Zeitpunkten und bei geringfügig höheren Cineol-Konzentrationen signifikant in ihrer Proliferation beeinflussen ließen. Diesem Unterschied liegt höchstwahrscheinlich auch die Tatsche zugrunde, dass es sich bei den *Polyposis nasi et sinuum*-Proben um Primärmaterial handelte und die Zellen durch die erforderlichen Versuchsschritte größerem Stress ausgesetzt waren, zusätzlich zu den zellspezifischen Charakteristika.

Jedoch unterstreichen diese Untersuchungen deutlich die beobachtete massive Proliferations-hemmende Wirkung von Cineol ab einer Konzentration von 1mM bis 2mM und einer deutlich zytotoxischen Wirkung bei 10mM in allen untersuchten Zellspezies.

Um die beobachteten Auswirkungen von Cineol eindeutig mit einer möglichen Apoptoseinduktion, einer Nekrose oder aber einer Zellzyklusarretierung in Zusammenhang zu bringen, werden die verschiedenen Zellen zur Zeit mittels Durchflusszytometer in An- und Abwesenheit der verschiedenen Cineol-Konzentrationen untersucht.

Die geschieht mittels Annexin-V und Propidiumiodid (PI) Färbungen und kann anschließend im FACS (Durchflusszytometer) quantitativ ausgewertet werden, wie in den ersten Vorversuchen deutlich zu sehen ist.

Mit der Annexin V-GFP/PI-FACS-Analyse können die einzelnen Populationen quantitativ detektiert werden. Die vitalen Zellen sind Annexin V-GFP/ PI negativ, die apoptotischen Zellen weisen eine Annexin V-Färbung auf. Zellen, welche schon in die sekundäre Nekrose übergegangen sind, sind Annexin V-GFP/ PI-positiv.

Während der Apoptose reichert sich in der äußeren Zellmembran Phosphatidylserin (PS) an, welches sich normalerweise nur in der inneren Schicht der Plasmamembran befindet. PS ist ein phagozytotisches Erkennungssignal, welches phagozytotische Zellen, wie Makrophagen, für die Entfernung der apoptotischen Zellen rekrutiert. Der sogenannte Totfarbstoff Propidiumiodid (PI) ist für die Membran impermeabel und kann die DNA nur in Zellen mit einer geschädigten Membran anfärben. Daher wird PI auch zur lebend/tot Unterscheidung verwendet.

### Einfluss von Cineol auf die zelluläre Migrationsaktivität

Neben der zellulären Vitalität und Progression sowie dem Einfluss auf Apoptose und Zellzyklus hat die Anmelderin auch den Einfluss von Cineol auf das zelluläre Migrationsverhalten untersucht. Aufgrund der Sensibilität der *Polyposis nasi et sinuum* Primärproben und der erforderlichen Reproduzierbarkeit der Versuchsanordnungen hat die Anmelderin in ihren Migrationsanalysen permanente Zelllinien maligner Kopf-Hals-Tumoren mit verschiedenen Konzentrationen von Cineol kultiviert und hinsichtlich ihrer Migrationsaktivität untersucht.

Die Versuche wurden mittels des X-Celligence Systems durchgeführt und zeigen lediglich einen leichten Einfluss von Cineol auf die zelluläre Migrationsaktivität bei einer Konzentration von 2 mM.

### Einfluss von Cineol auf den Transkriptionsfaktor NF-kB

Es wurde gezeigt, dass ein Inhalieren von Cineol im "Meerschweinchen-Modell" zu einer deutlichen Reduktion proinflamatorischer Zytokine führt, wobei jedoch die zugrundeliegenden molekularen Mechanismen nach wie vor unaufgeklärt sind.

Da auch für *Polyposis nasi et sinuum* postuliert wird, dass Entzündungsparameter einen entscheidenden Einfluss auf die Progression dieser Erkrankung haben, untersuchte die Anmelderin den Einfluss von Cineol auf die Aktivität des Transkriptionsfaktors Nuclear Factor kappa B (NF-kB) und auf die Aktivität eines breiten Spektrums verschiedener Phosphokinasen, um so die molekularen Angriffspunkte von Cineol in verschiedenen Biosynthesewegen der Zelle hinsichtlich des *Polyposis nasi et sinuum*-"Mikromilieus" zu identifizieren.

Der Transkriptionsfaktor NF-kB wurde 1986 im Labor von David Baltimore als ein Regulator der Expression des Gens für die kappa leichte Kette (kL) des Immunglobulins in murinen B-Lymphozyten entdeckt. Mittlerweile weiß man, dass dieser Transkriptionsfaktor ubiquitär vorkommt und als zentraler Koordinator eines breiten Spektrums unterschiedlichster zellulärer Funktionen fungiert. Vieles deutet darauf hin, dass dem Transkriptionsfaktor NF-κB auch eine Schlüsselfunktion bei der Tumorentstehung infolge chronischer Entzündungen zukommt. Die Funktionen von NF-κB sind jedoch sehr vielfältig und auch zellspezifisch. So kann die Inhibierung von NF-κB sowohl Apoptose bewirken als auch zur spontanen Tumorenstehung führen. All diese komplexen Signaltransduktionswege erfordern eine gut abgestimmte Regulation, so dass Dysregulationen von Transkriptionsfaktoren wie NF-κB maßgeblich in verschiedenen zellulären Aspekten der Onkogenese involviert sind. In vielen Zellen aktiviert NF-kB die Expression einer Vielzahl von inflammatorischen Zytokine, wie etwa IL-1 und IL-6, Chemokine wie IL-8, immunregulatorische Oberflächenmoleküle oder Zelladhäsionsmoleküle.

Die Untersuchungen zur Einflussnahme von Cineol auf die Aktivität des Transkriptionsfaktors NF-kB in *Polyposis nasi et sinuum* zeigen deutlich, dass Cineol keinen inhibierenden Einfluss hierauf hat, sondern vielmehr leicht aktivierend wirkt. Bei den Messergebnissen wurde TNF-alpha (tumor necrosis factor-alpha) als etablierter Stimulus der NF-kB Aktivität und somit als interne Positivkontrolle verwendet.

### Phosphokinase-Profile in Polyposis nasi et sinuum

Die Phosphorylierungslevel und somit die Aktivierung eines breiten Spektrums unterschiedlicher Phosphokinasen aus verschiedensten Biosynthesewegen wurde mittels eines "Phospokinase-Protein-Arrays" durchgeführt. Dieser Array bestimmt die Expressionsprofile von 44 phosphorylierten Phosphokinasen und erlaubt somit Rückschlüsse auf die Regulation unterschiedlicher Signaltransduktionskaskaden.

Das Prinzip dieses Protein-Arrays beruht auf der gleichzeitigen Analyse verschiedener Proteine auf einer Nitrozellulosemembran. Auf dieser Nitrozellulosemembran sind Primärantiköper ("Capture Antibodies") gegen die zu untersuchenden Proteine und Kontroll-Antikörper jeweils als Doppelbestimmungen gespottet worden. Nach der Inkubation mit gewonnenen Zelllysaten aus *Polyposis nasi et sinuum*-Gewebe wurden die ungebundenen Proteine durch entsprechende Waschschritte entfernt. Anschließend erfolgte die Inkubation mit einem spezifischen biotinyliertem sekundär-Antikörper, mit anschließender Inkubation mit einem Anti-Biotin-AP (Alkalische Phosphatase), wodurch die spezifisch an die Membran gebundenen Proteine sichtbar und somit quantifizierbar gemacht wurden.

Diese Untersuchungen wurden jeweils mit *Polyposis nasi et sinuum*-Gewebe im direkten Abgleich mit "gesunder" Mucosa desselben Patienten durchgeführt. Die erhaltenen Quotienten, also die Unterschiede zwischen Gesund und Krank, der durchgeführten Quantifizierungsergebnisse sind in Tabelle 1 dargestellt.

**Tabelle 1: Phosphokinase-Aktivitätsprofile.**

| | MW Polyp | MW Muschel | Stabw Polyp | Stabw Muschel | Quotient Pol/Muschel |
|---|---|---|---|---|---|
| Fyn;Y420 | 3,73 | 2,42 | 0,86 | 0,71 | 1,54 |
| Lyn;Y397 | 3,53 | 2,10 | 0,77 | 0,83 | 1,68 |
| c-jun;S63 | 6,61 | 4,51 | 1,38 | 0,40 | 1,47 |
| Pyk2;Y402 | 4,43 | 2,82 | 1,33 | 0,28 | 1,57 |
| STAT1;Y701 | 5,17 | 6,62 | 1,39 | 3,70 | 0,78 |
| STAT2;Y689 | 10,50 | 7,66 | 1,67 | 1,43 | 1,37 |
| STAT3;Y705 | 3,73 | 1,80 | 1,00 | 0,85 | 2,08 |
| STAT4;Y693 | 5,23 | 6,46 | 1,01 | 2,76 | 0,81 |
| STAT5a/b;Y699 | 5,59 | 5,50 | 1,44 | 2,29 | 1,02 |
| STAT5a;Y699 | 3,43 | 2,90 | 0,80 | 0,60 | 1,18 |
| STAT5b;Y699 | 14,61 | 7,60 | 7,19 | 4,42 | 1,92 |
| STAT6;Y641 | 7,76 | 6,09 | 2,30 | 2,21 | 1,28 |
| TOR;S2448 | 4,38 | 3,14 | 1,44 | 1,87 | 1,40 |
| CREB;S133 | 2,88 | 1,29 | 3,55 | 0,91 | 2,24 |
| GSK-3α/β;S21/S9 | 11,19 | 3,01 | 5,85 | 1,46 | 3,72 |
| β-Catenin;- | 2,88 | 2,24 | 1,32 | 0,95 | 1,29 |
| MEK1/2; | 5,01 | 2,80 | 1,29 | 1,04 | 1,79 |
| MSK1/2;S376/S360 | 4,75 | 3,61 | 0,88 | 1,11 | 1,31 |
| ERK1/2; T202/Y204, T185/Y187 | 38,59 | 16,50 | 46,47 | 23,69 | 2,34 |
| p27;T198 | 1,29 | 3,25 | 1,35 | 4,51 | 0,40 |
| p27;T157 | 1,63 | 2,15 | 1,06 | 0,77 | 0,76 |
| p38α | 2,38 | 1,93 | 1,58 | 1,17 | 1,23 |
| p53;S46 | 5,71 | 5,51 | 0,91 | 1,32 | 1,04 |
| p53;S392 | 1,44 | 2,00 | 1,14 | 0,98 | 0,72 |
| p53;S15 | 9,28 | 6,97 | 1,80 | 2,49 | 1,33 |
| Paxillin;Y118 | 4,47 | 2,99 | 1,15 | 0,63 | 1,50 |
| eNOS;S1177 | 6,59 | 3,00 | 4,50 | 2,75 | 2,20 |

Bei 5 Patienten mit chronisch-polypöser Sinusitis wurden Gewebeproben der Polypen und der unteren Nasenmuschel, die als interne Kontrolle dienten, mittels des Protein-Arrays untersucht.

In den untersuchten Gewebeproben der Nasenpolypen waren deutliche Verschiebungen im Vergleich zu gesunder Mucosa zu erkennen. So war beispielsweise der Phosphorylierungslevel des Proteins STAT3; Y705 in allen Proben signifikant erhöht. Das gleiche gilt für STAT5b, Y699 und GSK-3-alpha (siehe Tabelle 1).

Die Untersuchungen bilden die Grundlage weiterführender Analysen zur Identifizierung der für die Proliferation bei der *Polyposis nasi et sinuum* verantwortlichen Biosynthesewege.

So spielt etwa STAT3 eine zentrale Rolle im Pathway JAK-STAT. Die Aktivierung erfolgt z. B. durch die Bindung von IL-6 an den JAK-Rezeptor, daraus resultiert die Phosphorylierung von STATs. Diese dimerisieren und wandern in den Zellkern, wo sie die Transkription verschiedenster Zielgene im Rahmen der Zellproliferation aktivieren.

Die Anmelderin wird ihre Untersuchungen auf die funktionelle Charakterisierung dieser Proteine im Hinblick auf die Progression von *Polyposis nasi et sinuum* und die Wirkungsweise von Cineol fokussieren.

### mRNA-Expressionsprofile eines breiten Spektrum potentieller Stammzellmarker in Polyposis nasi et sinuum

Die chronisch-proliferative Natur der *Polyposis nasi et sinuum* und die hohe Rezidivrate auch nach operativer Sanierung der Nasennebenhöhlen lässt deutliche Parallelen zum Wachstum von Tumorerkrankungen erkennen. Bislang ist ungeklärt aus welchem Zellpool sich die Gewebszellen der nasalen Polypen rekrutieren, welche Signaltransduktionsprozesse für das Wachstum der Polypen eine Rolle spielen und aus welchem Grunde die nasalen Polypen nicht entarten.

Möglicherweise bilden Stammzellen einen zur chronischen Proliferation der nasalen Polypen beitragenden Zellpool, denn in neueren Studien konnte gezeigt werden, dass Stammzellen nicht nur bei der Entstehung und Differenzierung komplexer Organismen und bei Prozessen der Zellerneuerung eine essentielle Rolle spielen, sondern auch an der Entstehung und Progression von proliferativen Erkrankungen beteiligt sind. Möglicherweise könnten Stammzellen auch für die Pathophysiologie der *Polyposis nasi et sinuum* eine Rolle spielen.

Zur weiterführenden und genomweiten Identifizierung und Charakterisierung potentieller Stammzellpopulationen bei *Polyposis nasi et sinuum* wurde die Expression von Stammzellmarkern aus Einzellzellsuspensionen mittels Realtime PCR-Analysen untersucht.

Darüber hinaus wurde in weiteren Untersuchungen *Polyposis nasi et sinuum* Gewebe, sowie Gewebe der unteren Nasenmuschel mittels Microarrays (genome wide und spezielle Setups) vergleichend analysiert und auf Unterschiede in den Expressionsmustern hin untersucht.

Es wurden mRNA-Expressionsprofile potentieller Stammzellmarker in *Polyposis nasi et sinuum* erstellt. Die Auswertung dieser Untersuchungen ist noch nicht abgeschlossen, da weitere Daten zusätzlicher *Polyposis nasi et sinuum* Gewebeproben erhoben werden und derzeit mittels Microarray ermittelt werden. Bislang zeigt sich jedoch, dass die Expressionsprofile der verschiedenen Patienten sehr eigene Charakteristika aufweisen und sich trotz dieser individuellen Divergenzen einige signifikante Profilverschiebungen zwischen *Polyposis nasi et sinuum* und gesunder Mucosa abzeichnen, wie etwa die Transkriptionsfaktoren Nanog und FOXC2 oder der Migrationsmarker CXCR4.

Nach abschließender Auswertung der momentan laufenden Microarrays werden die daraus gewonnenen Ergebnisse auf Proteinebene verifiziert und dann hinsichtlich einer Einflussnahme durch verschiedene Cineol-Konzentrationen untersucht.

### Untersuchungen zum Wnt/FZD-Signalweg

Der Wnt-Frizzled-Signalweg spielt eine wichtige Rolle für regulative Prozesse der Embryonalentwicklung, den Erhalt der Stammzellreservoire sich selbstregenerierender Organe und die Aktivierung der Proliferation von Stammzellen.

Die Grundlagen des Wnt-Signalweges wurde in den späten 1980er bzw. frühen 1990er Jahren erforscht, als gezeigt werden konnte, dass die Genprodukte von *Drosophila wingless* (wg) und Maus Int1 (später in Wnt1 umbenannt) zu einer großen, evolutionär erhaltenen Familie extrazellulärer Signalmoleküle gehören .

Wnt-Proteine sind Glykoprotein-Liganden der Frizzled (FZD)-Transmembranrezeptorfamilie, von denen beim Menschen bis heute 19 bekannt sind. Abhängig vom Rezeptor-Kontext, Zelltyp und dem Differenzierungsstadium der Zelle können die Wnt-Liganden mindestens drei verschiedene intrazelluläre Signalkaskaden initiieren, welche in der Transkriptionsregulation verschiedener Gene mündet. Es werden voneinander der "*Canonical*"*-,* der "*Wnt*/*Planar cell polarity*"*-* und der "*Wnt-Ca*²⁺"-Signalweg unterschieden. Bei der "*Canonical*"-Signalkaskade führt die Interaktion von Wnt-Liganden (in der Regel Wnt3a) mit den FZD-Rezeptoren unter Mitwirkung mit dem "*low density lipoprotein receptor-related protein*" *(LRP)* zur Inhibition der Glykogen-Synthase-Kinase-3-beta (GSK-3-beta), in dessen Folge es zur Stabilisierung und Akkumulation von beta-Catenin im Zytoplasma, dessen Translokation in den Zellkern und der Transkription TCF4/LEF-abhängiger Gene kommt. Liegt eine Wnt3a/FZD-Interaktion nicht vor, so wird beta-Catenin durch den Axin/GSK3-beta/APC-Komplex phosphoryliert und in dessen Folge nach Ubiquitinylierung proteasomvermittelt abgebaut. Bei dem beta-Cateninunabhängigem "non-canonical"-Wnt/Ca²⁺-Signalweg kommt es nach Interaktion von Wnt-Liganden (typischerweise Wnt5a) mit FZD-Rezeptoren (typischerweise FZD2/FZD5) über Phospholipase C zu einem Ca²⁺-Einstrom und Aktivierung Ca²⁺-abhängiger Faktoren, wie Proteinkinase C (PKC) und Ca²⁺/Calmodulin-dependent Kinase II (CaMKII). Hieraus kann eine Transkriptionsaktivität über die Tanskriptionsfaktoren NF-kappaB, NFAT und die AP1-Familie resultieren.

Der Wnt/FZD-Signalweg kann über zwei Klassen von extrazellulären Antagonisten gehemmt werden, welche beide die Interaktion zwischen Ligand und Rezeptor unterbinden: Die erste Klasse bilden die *sFRP* "*secreted Frizzled-related protein*"*,* "*Wnt-inhibitory-factor-1*" und *Cerberus,* welche Wnt-Proteine binden und somit deren Bindung an Wnt-Rezeptoren unterbinden. Die zweite Klasse der Wnt-Antagonisten bilden die "*Dickkopf*" *(DKK)-*Proteine, welche eine Blockierung des Co-Rezeptors LRP5/LRP6 bewirken können.

Die Komplexität der Wnt/FZD-Signaltransduktionswege erfordern eine gut abgestimmte Regulation. Eine Dysregulationen bzw. Überexpression von Wnt-Signaling kann ungerichtetes Wachstum unterschiedlicher Zellen mitverursachen. Beim Kolonkarzinom finden sich Mutationen der im Wnt-Signalweg involvierten Proteine APC und Axin. Ferner wurde für zahlreiche Tumorentitäten ein epigenetisches "Silencing" von Wnt-Inhibitory factor-1 gezeigt, welches eine Deregulation des Wnt/FZD-Signalweges begünstigen könnte.

Seit den ersten Berichten einer Wnt5a-vermittelten Überexpression von Zytokinen und proinflammatorischen Zytokinen in Fibroblasten bei der rheumatoiden Arthritis konnte eine Rolle des Wnt/FZD-Signalweges für die Regulation entzündlicher Prozesse bei Psoriasis, Sepsis, Asthma und Tuberkulose gezeigt werden. Für endotheliale Zellen wurde beschrieben, dass über den beta-Catenin-unabhängigen Wnt/Ca²⁺-Signalweg die Expression von COX-2 induziert und die Zytokinausschüttung verstärkt werden kann. Im Gegensatz zum Wnt/Ca²⁺-Signalweg scheint das beta-Catenin-abhängige "canonical"-Wnt-Signaling eine immunmodulierende bzw. antiinflammatorische Rolle einzunehmen. Hierauf deuten kürzlich erzielte Ergebnisse hin, welche bei Makrophagen eine verminderte TNF-alpha-Expression nach Aktivierung des Wnt/beta-Catenin-Signalings durch Wnt3a bzw. einem GSK-3-beta-Inhibitor zeigen konnten. In einer anderen Studie führte die Inhibition der beta-Cateninexpression in dendritischen Zellen zu einer Verstärkung der Entzündungsantwort, womit die Rolle von beta-Catenin für eine Dämpfung der Immunantwort nachdrücklich demonstriert werden konnte. Die beta-Cateninexpression intestinaler dendritischer Zellen scheint ferner maßgeblich für die Balance des Aktivierungstatus von regulatorischen T-Zellen und T-Effektorzellen zu sein.

Zahlreiche Medikamente nehmen Einfluss auf die Wnt-Expression. Interessanterweise verstärken Glukokortikosteroide die Expression von Wnt-Antagonisten und können somit das Wnt-Signaling unterdrücken. Somit besteht eine unmittelbare Interaktion zwischen dem aktuell wichtigsten Pharmakon bei CRSwP, den Glukokortikosteroiden, mit dem Wnt-Signalweg. Für Acetylsalicylsäure (ASS), welche über einen bislang unbekannten Mechanismus eine wichtige Rolle für die Entwicklung einer CRSwP bei Patienten mit ASS-Intoleranz spielt, konnte in einer Genexpressionsanalyse von Colon-Ca-Zelllinien eine Hochregulation von WIF-1 und somit eine Wnt-Suppression gezeigt werden.

Die anti- und proinflammatorischen Eigenschaften der "canonischen" (Wnt/beta-Catenin-Signaling) und "nicht-canonischen" Wnt-Signalwege scheint bei der chronischen Rhinosinusitis mit Polyposis (CRSwP) eine wichtige Rolle für die Modulation akuter und chronischer Entzündungsprozesse einzunehmen. Ein besseres Verständnis des Wnt/FZD-Signalweges bei CRSwP kann zu neuartigen Therapieansätzen und somit zu einer Reduktion der Rezidivhäufigkeit und der Notwendigkeit operativer Eingriffe bei der CRSwP beitragen.

Im Folgenden sind die bisherigen, viel versprechenden Ergebnisse zum Wnt/FZD-Signaling bei CRSwP aufgeführt und erläutert.

Durch Microarrayanalysen (Agilent Whole Human Genome Oligo) hat die Anmelderin umfassende Genexpressionsdaten von 5 Pärchen Polyp/untere Nasenmuschel von 5 Patienten mit CRSwP erhalten.

### Wnt Inhibitory factor-1 (WIF-1)

Bei der Analyse der Microarraydaten fiel in allen Präparaten eine hochsignifikante, zum Teil 100-fache Herabregulation von Wnt-Inhibitory-factor-1 (WIF-1) auf, welches sich in der RT-PCR (TaqMan Assay) mit einer 4- bis 230-fachen Herabregulation bestätigen ließ.

Für die weiteren Wnt-Inhibitoren wie DKK (Dickkopf) und SFRP (Secreted Frizzled Related Protein) zeigte sich lediglich für SFRP 5 eine signifikante (p < 0,01) Herabregulation. Die WIF-1-Immunhistochemie zeigte im Nasenmuschelgewebe Immunpositivität im Bereich des subepithelialen Stromas, während das Epithel wenig bis keine WIF-1-Immunpositivität aufwies. Im Polyposisgewebe zeigte sich nur eine schwache Immunpositivität.

Eine Herabregulation von Wnt-Inhibitoren kann eine Deregulation des Wnt/FZD-Signalweges zur Folge haben und könnte somit zur Entzündung und Progression CRSwP beitragen.

In der Fluoreszenz-Immunhistochemie gegen WIF-1 zeigte sich im Nasenmuschelgewebe Immunpositivität im Bereich des subepithelialen Stromas, während das Epithel wenig bis keine WIF-1-Immunpositivität aufwies. Das Polyposisgewebe zeigte nur subepithelial vereinzelte, gering immunpositive Areale.

Ein KEGG-Diagramm des Wnt/FZD-Signalweges bei CRSwP wurde erstellt. Die Microarraydaten wurden mit der Software "Onto-Tools" in das KEGG-Diagramm überführt und ausgewertet.

### Wnt-Proteine

Der Vergleich von Polypen und unteren Nasenmuscheln auf Grundlage der Microarrayanalyse ergab (nach "poolen" der Ergebnisse und Fehlerkorrektur mittels Rosetta-Fehlermodell; Rosetta Resolver Software) eine signifikante Hochregulation mehrerer Wnt-Proteine.

Die Microarraydaten der 5 Polypen und 5 Nasenmuscheln wurden gepoolt und nach Fehlerkorrektor (Rosetta-Software) miteinander verglichen. Es wurden die Expressionsunterschiede von Polyp versus Nasenmuschel ausgewertet. Es erfolgte eine Bestätigung der Microarraydaten über RT-qPCR (TaqMan Assay). Bis auf Wnt7a zeigte sich eine gute Korrelation der Microarraydaten mit den RT-qPCR-Daten

Neben einer signifikanten Hochregulation des für die Aktivierung des Wnt/beta-Catenin-Pathway verantwortlichen Wnt3a zeigte sich deutlich erhöhte mRNA-Level von Wnt4 und Wnt7a, deren Rolle für das Wnt/FZD-Signaling bislang nur wenig charakterisiert sind.

Die mRNA-Expressionslevel der zwischen Polyp und unterer Nasenmuschel signifikant (p < 0,05) erhöhten oder erniedrigten Wnt-Proteine konnten über RT-qPCR (Taq-Man Assays) weitgehend bestätigt werden. Immunhistochemisch konnte die Anmelderin bislang Wnt3a und Wnt4 nachweisen. Die LSAB-Immunhistochemie für Wnt3A und Wnt4 zeigte im Polyposisgewebe Immunpositivität sowohl des Epithels als auch von Fibroblasten und entzündlichem Infiltrat.

Der Proteinnachweis von Wnt5a ist der Anmelderin mit den bislang verwendeten Methoden (Western Blot und Immunhistochemie) noch nicht gelungen und soll deshalb demnächst über Immunpräzipitation erfolgen. Unabhängig vom Präparat gilt jedoch die Nachweisbarkeit von Wnt5a auf Proteinebene generell als technisch anspruchsvoll.

### Frizzled (FZD)-Rezeptoren

Die Microarrayanalyse ergab ein heterogenes Bild herauf- und herabregulierter die Frizzled(FZD)-Rezeptoren, d. h. die Microarrayergebnisse zeigten ein heterogenes Bild von herauf- und herabregulierten FZD-Rezeptoren. Auffällig ist die > 3-fache Heraufregulation von FZD2, einem Aktivator des Wnt/Ca²⁺-Signalings

Die Expressionslevel der FZD2-mRNA war 3,53-fache (Microarray) bzw. 2,21-fache (RT-qPCR) hochreguliert. Über FZD2 wie auch FZD5 kann der Wnt/Ca²⁺-Signalweg aktiviert werden, über welchen unter anderem durch Makrophagenaktivierung proinflammatorische Prozesse aktiviert werden können. Die FZD2-Immunhistochemie zeigte eine ausgeprägte Fluoreszenzintensität des Epithels der Polypen. Im Western-Blot konnte die in Microarray und RT-PCR gefundene verstärkte Expression von FZD2 im Polyp im Vergleich zur unteren Nasenmuschel bestätigt werden.

Ein Vergleich der FZD2-mRNA-Level von Polyp versus Nasenmuschel erfolgte mittels Microarray (MA) und RT-qPCR (PCR).

Die FZD2-Immunhistochemie zeigte eine ausgeprägte Fluoreszenzintensität des Epithels der Polypen. Im Western-Blot konnte die in Microarray und RT-PCR gefundene verstärkte Expression von FZD2 im Polyp im Vergleich zur unteren Nasenmuschel bestätigt werden.

### Wnt/beta-Caten in-Signaling

Im entzündlichen Infiltrat des Polyposisgewebes ließ sich immunhistochemisch nukleäres beta-Catenin nachweisen. Nach Inkubation von Polyposis-Einzelzellsuspensionen in Chamber Slides mit dem GSK-3-beta-Inhibitor SB216763 zeigte sich in der anti-beta-Catenin-Immunhistochemie eine Akkumulation von beta-Catenin im Zytoplasma und eine Translokation von beta-Catenin in den Zellkern.

Es wurde eine Anti-Active-beta-Catenin-Immunhistochemie auf mit dem GSK-3-Inhibitor SB216763 für 24 h inkubierter Polyposis-Einzelzellsuspension (Chamber Slide) durchgeführt. Durch Inhibition des beta-Catenin-Abbaus zeigt sich eine deutliche Anreicherung von beta-Catenin sowie eine Translokation von beta-Catenin in den Zellkern.

Zur funktionellen Untersuchung der Rolle des Wnt/beta-Catenin-Signalings auf die Zytokinexpression, wurde Polyposis-Primärgewebe mit den Wnt/beta-Catenin-Signalingaktivatoren Wnt3a und SB216763 inkubiert. Übereinstimmend mit anderen Ergebnissen einer verminderten Entzündungsantwort bei Makrophagen nach Inkubation mit Wnt3a bzw. dem GSK3-beta-Inhibitor SB216763 zeigte sich im Polyposisgewebe eine verminderte Ausschüttung von IL-6 und IL-8.

### Zusammenfassung der Untersuchungen

Folgende Erkenntnisse der Untersuchungen lassen sich zusammenfassen:
- Einfluss von Cineol auf Zellviabilität und Progression auf benigne und maligne Zellspezies: Analyse der Zellzykluscharakteristika und der Mechanismen einer möglichen Apoptoseregulation.
- Einfluss von Cineol auf die zelluläre Migrationsaktivität: Genomweite Microarray-Analyse der *Polyposis nasi et sinuum* mRNA-Expressionsmuster unter dem Einfluss von Cineol und eine anschließende Auswertung hinsichtlich Migrations-relevanter Parameter.
- Einfluss von Cineol auf NF-kB- und Phosphokinase-Aktivitätsprofile: Verifizierung der identifizierten Phosphokinasen auf Proteinebene und anschließende funktionelle Charakterisierung.
- Einfluss von Cineol auf die Expressionsprofile potentieller Stammzellmarker: Verifizierung der identifizierten Stammzellmarker auf Proteinebene und anschließende funktionelle Charakterisierung.

### Weitere klinische Anwendungen

Zusammenfassend kann also im Rahmen der vorliegenden Erfindung erstmals ein neuartiges, zumindest im Wesentlichen nebenwirkungsfreies Therapiekonzept für die prophylaktische und/oder therapeutische Behandlung von Nasenpolypen *(Polyposis nasi et sinuum),* insbesondere auch rezidivierenden Formen, auf Basis eines naturbasierten Wirkstoffs (Cineol. Insbesondere 1,8-Cineol) bereitgestellt werden, mit welchem die Nachteile des Standes der Technik zumindest weitgehend vermieden oder aber wenigstens abgeschwächt werden können und mit welchem sich vergleichbare, bevorzugt sogar eine verbesserte Wirkeffizienz im Vergleich zu den Therapiekonzepten des Standes der Technik erreichen lassen.

Zur weitergehenden klinischen Verifizierung des erfindungsgemäßen Konzepts werden weitere klinische Daten ermittelt. Insbesondere kann die Therapiewirkung von Cineol, insbesondere 1,8-Cineol, auch im Rahmen der chronischen polypösen Sinusitis mit *Polyposis nasi et sinuum* verifiziert werden (randomisierte Doppel-Blind- und placebokontrollierte Studie). Ziel des erfindungsgemäßen Therapiekonzepts ist insbesondere auch eine anhaltende Rezidivfreiheit bzw. zumindest eine Verlängerung des rezidivfreien Intervalls, insbesondere infolge der antientzündlichen und kortikosteroidartigen Wirkung von Cineol, insbesondere 1,8-Cineol. Auch kommt es zu einer Vermeidung oder zumindest Reduktion der bekannten Dauertherapie mit Kortikosteroiden (Glucokortikoiden), insbesondere auch bei gleichzeitig bestehendem *Asthma bronchiale.*

### Einschlusskriterien

Klinisch (endoskopisch/radiologisch/CT) gesicherte chronisch polypöse Pansinusitis mit oder ohne *Polyposis nasi*, ggf. auch Rezidiverkrankung, ggf. auf Basis eines *Asthma bronchiale*
Letzte systematische Kortikosteroid-Therapie vor 6 Wochen
Keine Therapie mit Cineol in den letzten 6 Wochen
Keine Therapiewechsel in den letzten 4 Wochen vor Studienbeginn
Keine Unverträglichkeit gegenüber ätherischen Ölen
Keine bakteriell entzündlichen Atemwegserkrankungen in den letzten 4 Wochen
Regelmäßiges Follow-up möglich
Keine Schwangerschaft oder Stillzeit
Keine schwere kardiopulmonale Begleiterkrankung
Keine Malignome Erkrankung
Keine andere chronische Dauererkrankung (unkontrollierter Diabetes; rheumatoide Arthritis)

### Unterteilung der Patienten in 3 Gruppen:

1. Kontrollgruppe mit herkömmlicher nasaler Mometason-Verabreichung
2. Nasale Mometason-Verabreichung + systemische Gabe von Cineol
3. Nasale Mometason-Verabreichung + topische Gabe von Cineol

### Untersuchungen:

1. prätherapeutisch:
2. präoperativ (PE intraoperativ)
3. 3 Wochen postoperativ
4. 6 Wochen postoperativ
5. 12 Wochen postoperativ
Klinische HNO-Ärztliche Untersuchung
Endoskopie (0°, 30°, 70° Optik)
Allergietest
Rhinomanometrie
Olfaktometrie
Computertomogramm der Nasennebenhöhlen
ggf. Lungenfunktion/Bodyplethysmographie
PE aus der Polyposis; ggf. auch mittlere und/oder untere Nasenmuschel möglichst bei allen Kontrollen
Blutentnahme
Fragebogen zur Lebensqualität entsprechend der Kontrollen
Follow-up
"Tagebuch" des Patienten
Cineol-Microarraystudie
Untersuchung der Genexpression bei chronischer Rhinosinusitis mit Polyposis (CRSwP) unter 1,8-Cineol-Therapie

### Einschlusskriterien:

Chronische Rhinosinusitis mit *Polyposis nasi et sinuum* (CRSwP)

### Ausschlusskriterien:

systemische oder topische Glukokortikoid-Therapie vor weniger als 6 Wochen Unverträglichkeit gegenüber ätherischen Ölen oder Sorbitol
Bakterieller Infekt der oberen Atemwege
Antibiotikatherapie
Malignome Erkrankung
Asthma bronchiale
Ulcus duodeni
Schwangerschaft oder Stillzeit
Allergie

### Experimente:

Entnahme von Polyposis-nasi-Gewebe bei ca. 10 Patienten vor Cineolgabe und nach ca. einwöchiger Cineoltherapie (intraoperativ).
Entnahme von Nasenmuschelgewebe von ca. 10 "gesunden" Patienten (keine CRS, keine weitere Medikation, keine Allergie) während Septumplastik mit Conchotomie
Vergleich der Genexpression des prä- und intraoperativ entnommenen Polyposisgewebes intraindividuell sowie interindividuell mit dem "gesunden" Nasenmuschelgewebe durch Microarray-Analyse.
Bestätigung der Microarray-Analyse durch RT-qPCR, Immunhistochemie und Western-Blot.

### Erforderliche Patientendaten:

Zeitpunkt der letzten Glukokortikoidtherapie
Alter
Geschlecht
Allergien / ASS-Intoleranz
Medikamente
Nebenerkrankungen

## Patentansprüche

1. Zusammensetzung, insbesondere pharmazeutische Zusammensetzung, zur Verwendung bei der prophylaktischen und/oder therapeutischen Behandlung von Nasenpolypen *(Polyposis nasi et sinuum),*
wobei die Zusammensetzung als Wirkstoff, insbesondere pharmazeutischen Wirkstoff, Cineol, vorzugsweise 1,8-Cineol, enthält.

2. Zusammensetzung zur Verwendung nach Anspruch 1,
wobei die Zusammensetzung zur prophylaktischen und/oder therapeutischen Behandlung von rezidivierenden Formen von Nasenpolypen *(Polyposis nasi et sinuum)* eingesetzt wird.

3. Zusammensetzung zur Verwendung nach Anspruch 1 oder 2,
wobei die Zusammensetzung zur prophylaktischen und/oder therapeutischen Behandlung von mit dem Wnt-Signalweg, insbesondere Wnt/beta-Catenin-Signaltransduktionsweg, in Zusammenhang stehenden, vorzugsweise induzierten oder begünstigten Formen von Nasenpolypen *(Polyposis nasi et sinuum)* eingesetzt wird.

4. Zusammensetzung zur Verwendung nach einem der vorangehenden Ansprüche,
wobei die Zusammensetzung zur prophylaktischen und/oder therapeutischen Behandlung von postoperativen Zuständen eingesetzt wird.

5. Zusammensetzung zur Verwendung nach einem der vorangehenden Ansprüche,
wobei die Zusammensetzung zur prophylaktischen und/oder therapeutischen Behandlung von Nasenpolypen *(Polyposis nasi et sinuum),* insbesondere rezidivierenden Formen, nach vorangehender chirurgischer Entfernung der Nasenpolypen eingesetzt wird.

6. Zusammensetzung zur Verwendung nach einem der vorangehenden Ansprüche,
wobei die Zusammensetzung zur Verringerung des Risikos oder Wahrscheinlichkeit der Rezidivbildung, insbesondere bei postoperativen Zuständen und/oder Zuständen nach vorangehender chirurgischer Entfernung der Nasenpolypen, eingesetzt wird.

7. Zusammensetzung zur Verwendung nach einem der vorangehenden Ansprüche,
wobei die Zusammensetzung zur Rezidivprophylaxe, insbesondere bei postoperativen Zuständen und/oder Zuständen nach vorangehender chirurgischer Entfernung der Nasenpolypen, eingesetzt wird.

8. Zusammensetzung zur Verwendung nach einem der vorangehenden Ansprüche,
wobei die Zusammensetzung das Cineol als Reinstoff mit einer Reinheit von mindestens 95 Gew.-%, bezogen auf das Cineol, und frei von anderen Terpenen enthält und
wobei die Zusammensetzung das Cineol als alleinigen Wirkstoff, insbesondere als alleinigen pharmazeutischen Wirkstoff, enthält.

9. Zusammensetzung zur Verwendung nach einem der vorangehenden Ansprüche,
wobei die Zusammensetzung das Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält

10. Zusammensetzung zur Verwendung nach einem der vorangehenden Ansprüche,
wobei das Cineol mit einer Tagesdosis im Bereich von 0,1 bis 5.000 mg/diem, insbesondere im Bereich von 1 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht wird.

11. Zusammensetzung zur Verwendung nach einem der vorangehenden Ansprüche,
wobei das Cineol systemisch mit einer Tagesdosis im Bereich von 10 bis 5.000 mg/diem, insbesondere im Bereich von 50 bis 3.000 mg/diem, vorzugsweise im Bereich von 100 bis 2.500 mg/diem, bevorzugt im Bereich von 150 bis 2.000 mg/diem, besonders bevorzugt im Bereich von 200 bis 1.500 mg/diem, verabreicht wird oder aber
wobei das Cineol topisch mit einer Tagesdosis im Bereich von 0,1 bis 2.000 mg/diem, insbesondere im Bereich von 0,5 bis 1.500 mg/diem, vorzugsweise im Bereich von 1 bis 1.000 mg/diem, bevorzugt im Bereich von 2 bis 1.500 mg/diem, besonders bevorzugt im Bereich von 5 bis 1.000 mg/diem, verabreicht wird.

12. Zusammensetzung zur Verwendung nach einem der vorangehenden Ansprüche,
wobei die Zusammensetzung als Kombinationstherapeutikum mit Kortikosteroiden, insbesondere systemischen oder topischen Kortikosteroiden, eingesetzt wird.

13. Arzneimittel zur Verwendung bei der prophylaktischen und/oder therapeutischen Behandlung von Nasenpolypen *(Polyposis nasi et sinuum),*
wobei das Arzneimittel als Wirkstoff, insbesondere pharmazeutischen Wirkstoff, Cineol, vorzugsweise 1,8-Cineol, enthält.

14. Wirkstoffkombination, insbesondere in Form eines Kit (Kit-of-parts), zur Verwendung bei der prophylaktischen und/oder therapeutischen Behandlung von Nasenpolypen (*Polyposis nasi et sinuum*),
wobei die Wirkstoffkombination als räumlich getrennte, aber funktional zusammenhängende und/oder zur Co-Medikation oder Kombinationstherapie bestimmte Komponenten
(a) Cineol, insbesondere 1,8-Cineol, bevorzugt in Form einer das Cineol enthaltenden, topisch oder systemisch zu applizierenden pharmazeutischen Zusammensetzung, einerseits und
(b) mindestens ein Kortikosteroid, bevorzugt in Form einer das Kortikosteroid enthaltenden, topisch oder systemisch zu applizierenden pharmazeutischen Zusammensetzung,
umfasst.

15. Cineol, insbesondere 1,8-Cineol, zur Verwendung bei der prophylaktischen und/oder therapeutischen Behandlung von mit dem Wnt-Signalweg, insbesondere Wnt/beta-Catenin-Signaltransduktionsweg, in Zusammenhang stehenden und/oder hierdurch induzierten und/oder begünstigten Nasenpolypen (*Polyposis nasi et sinuum*), vorzugsweise rezidivierenden Formen von Nasenpolypen (*Polyposis nasi et sinuum*);
wobei das Cineol als Regulator, vorzugsweise Inhibitor und/oder Suppressor, des Wnt-Signalwegs, insbesondere Wnt/beta-Catenin-Signaltransduktionswegs, vorzugsweise als Regulator und/oder Verringerer der Phosphorylierung der GSK-3-Proteinkinase im Rahmen des Wnt-Signalwegs oder zur Aktivierung der GSK-3-Proteinkinase und/oder als Deaktivator von beta-Catenin im Rahmen des Wnt-Signalwegs, eingesetzt wird; und/oder
wobei das Cineol als Regulator, vorzugsweise Inhibitor und/oder Suppressor, mindestens eines Wnt-Gens und/oder Wnt-Proteins eingesetzt wird.

## Claims

1. A composition, in particular pharmaceutical composition, for use in the prophylactic and/or therapeutic treatment of nose polyps (*polyposis nasi et sinuum*),
wherein the composition contains cineole, preferably 1,8-cineole, as the active ingredient, in particular as the pharmaceutical active ingredient.

2. The composition for use according to claim 1,
wherein the composition is used for the prophylactic and/or therapeutic treatment of recurrent forms of nose polyps (*polyposis nasi et sinuum*).

3. The composition for use according to claim 1 or 2,
wherein the composition is used for the prophylactic and/or therapeutic treatment of preferably induced or beneficiary forms of nose polyps (*polyposis nasi et sinuum*) associated with the Wnt signalling pathway, in particular Wnt/beta-catenin signal transduction pathway.

4. The composition for use according to any one of the preceding claims,
wherein the composition is used for the prophylactic and/or therapeutic treatment of postoperative conditions.

5. The composition for use according to any one of the preceding claims,
wherein the composition is used for the prophylactic and/or therapeutic treatment of nose polyps (*polyposis nasi et sinuum*), in particular recurrent forms, after previous surgical removal of nose polyps.

6. The composition for use according to any one of the preceding claims,
wherein the composition is used to reduce the risk or likelihood of recurrence, particularly in postoperative conditions and/or conditions following previous surgical removal of nose polyps.

7. The composition for use according to any one of the preceding claims,
wherein the composition is used for relapse prophylaxis, in particular in postoperative conditions and/or conditions after previous surgical removal of nose polyps.

8. The composition for use according to any one of the preceding claims,
wherein the composition contains cineole as a pure substance having a purity of at least 95% by weight, relative to the cineole, and is free of other terpenes, and
wherein the composition contains cineole as the sole active ingredient, in particular as the sole pharmaceutical active ingredient.

9. The composition for use according to any one of the preceding claims,
wherein the composition contains cineole, relative to the composition, in relative amounts in the range of 0.0001 to 80% by weight, in particular 0.001 to 75% by weight, preferably 0.005 to 70% by weight, more preferably 0.01 to 60% by weight, particularly preferably 0.05 to 55% by weight, very particularly preferably 0.1 to 50% by weight.

10. The composition for use according to any one of the preceding claims,
wherein the cineole is administered at a daily dose in the range from 0.1 to 5,000 mg/diem, in particular in the range from 1 to 3,000 mg/diem, preferably in the range from 5 to 2,500 mg/diem, preferably in the range from 10 to 2,000 mg/diem, particularly preferably in the range from 50 to 1,500 mg/diem.

11. The composition for use according to any one of the preceding claims,
wherein the cineole is systematically administered at a daily dose in the range from 10 to 5,000 mg/diem, in particular in the range from 50 to 3,000 mg/diem, preferably in the range from 100 to 2,500 mg/diem, preferably in the range from 150 to 2,000 mg/diem, particularly preferably in the range from 200 to 1,500 mg/diem or
wherein the cineole is topically administered at a daily dose in the range from 0.1 to 2,000 mg/diem, in particular in the range from 0.5 to 1,500 mg/diem, preferably in the range from 1 to 1,000 mg/diem, preferably in the range from 2 to 1,500 mg/diem, particularly preferably in the range from 5 to 1,000 mg/diem.

12. The composition for use according to any one of the preceding claims,
wherein the composition is used as a combination therapeutic agent with corticosteroids, in particular systemic or topical corticosteroids.

13. A medicinal product for use in the prophylactic and/or therapeutic treatment of nose polyps (*polyposis nasi et sinuum*),
wherein the medicinal product contains cineole, preferably 1,8-cineole, as the active ingredient, in particular as the pharmaceutical active ingredient.

14. An active ingredient combination, in particular in the form of a kit (kit-of-parts), for use in the prophylactic and/or therapeutic treatment of nose polyps (p*olyposis nasi et sinuum*),
wherein the active ingredient combination contains, as spatially separated but functionally connected components and/or components intended for co-medication or combination therapy,
(a) cineole, in particular 1,8-cineole, preferably in the form of a pharmaceutical composition containing cineole and to be applied topically or systemically, on the one hand, and
(b) at least one corticosteroid, preferably in the form of a pharmaceutical composition containing corticosteroid and to be applied topically or
systemically.

15. Cineole, in particular 1,8-cineole, for use in the prophylactic and/or therapeutic treatment of nose polyps, associated with the Wnt signalling pathway, in particular Wnt/beta-catenin signal transduction pathway, and/or induced thereby and/or beneficiary forms of nose polyps (*polyposis nasi et sinuum*), preferably recurrent forms of nose polyps (*polyposis nasi et sinuum*);
wherein the cineole is used as regulator, preferably an inhibitor and/or suppressor, of the Wnt signalling pathway, in particular Wnt/beta-catenin signal transduction pathway, preferably as a regulator and/or reducer of phosphorylation of the GSK-3 protein kinase within the Wnt signalling pathway or for activation of the GSK-3 protein kinase and/or as a deactivator of beta-catenin within the Wnt signalling pathway; and/or
wherein the cineole is used as a regulator, preferably an inhibitor and/or suppressor, of at least one Wnt gene and/or Wnt protein.

## Revendications

1. Composition, en particulier composition pharmaceutique, destinée à être utilisée dans le traitement prophylactique et/ou thérapeutique des polypes nasaux (polypose naso-sinusienne),
la composition contenant du cinéol, de préférence du 1,8-cinéol, comme principe actif, en particulier comme principe actif pharmaceutique.

2. Composition destinée à être utilisée selon la revendication 1,
la composition étant utilisée pour le traitement prophylactique et/ou thérapeutique de formes récidivantes de polypes nasaux (polypose naso-sinusienne).

3. Composition destinée à être utilisée selon la revendication 1 ou 2,
la composition étant utilisée pour le traitement prophylactique et/ou thérapeutique de formes de polypes nasaux (polypose naso-sinusienne) de préférence induites ou favorisées associées à la voie de signalisation Wnt, en particulier la voie de transduction du signal Wnt/bêta-caténine.

4. Composition destinée à être utilisée selon l'une quelconque des revendications précédentes,
la composition étant utilisée pour le traitement prophylactique et/ou thérapeutique d'états postopératoires.

5. Composition destinée à être utilisée selon l'une quelconque des revendications précédentes,
la composition étant utilisée pour le traitement prophylactique et/ou thérapeutique des polypes nasaux (polypose naso-sinusienne), en particulier des formes récidivantes, après ablation chirurgicale préalable des polypes nasaux.

6. Composition destinée à être utilisée selon l'une quelconque des revendications précédentes,
la composition étant utilisée pour réduire le risque ou la probabilité de récidive, en particulier dans des états postopératoires et/ou des états suivant l'ablation chirurgicale préalable des polypes nasaux.

7. Composition destinée à être utilisée selon l'une quelconque des revendications précédentes,
la composition étant utilisée pour la prophylaxie des récidives, en particulier dans des états postopératoires et/ou des états suivant l'ablation chirurgicale préalable des polypes nasaux.

8. Composition destinée à être utilisée selon l'une quelconque des revendications précédentes,
la composition contenant le cinéol comme substance pure ayant une pureté d'au moins 95 % en poids, par rapport au cinéol, et étant exempte d'autres terpènes, et
la composition contenant le cinéol comme seul principe actif, en particulier comme seul principe actif pharmaceutique.

9. Composition destinée à être utilisée selon l'une quelconque des revendications précédentes,
la composition contenant le cinéol, rapporté à la composition, en des quantités relatives situées dans la plage allant de 0,0001 à 80 % en poids, en particulier de 0,001 à 75 % en poids, de préférence de 0,005 à 70 % en poids, de manière plus préférée de 0,01 à 60 % en poids, de manière particulièrement préférée de 0,05 à 55 % en poids, de manière encore plus préférée de 0,1 à 50 % en poids.

10. Composition destinée à être utilisée selon l'une quelconque des revendications précédentes,
le cinéol étant administré à une dose quotidienne située dans la plage allant de 0,1 à 5000 mg/jour, en particulier dans la plage allant de 1 à 3000 mg/jour, de préférence dans la plage allant de 5 à 2500 mg/jour, de manière plus préférée dans la plage allant de 10 à 2000 mg/jour, de manière particulièrement préférée dans la plage allant de 50 à 1500 mg/jour.

11. Composition destinée à être utilisée selon l'une quelconque des revendications précédentes,
le cinéol étant administré par voie générale à une dose journalière située dans la plage allant de 10 à 5000 mg/jour, en particulier dans la plage allant de 50 à 3000 mg/jour, de préférence dans la plage allant de 100 à 2500 mg/jour, de manière plus préférée dans la plage allant de 150 à 2000 mg/jour, de manière particulièrement préférée dans la plage allant de 200 à 1500 mg/jour, ou
le cinéol étant administré par voie topique à une dose quotidienne située dans la plage allant de 0,1 à 2000 mg/jour, en particulier dans la plage allant de 0,5 à 1500 mg/jour, de préférence dans la plage allant de 1 à 1000 mg/jour, de manière plus préférée dans la plage allant de 2 à 1500 mg/jour, de manière particulièrement préférée dans la plage allant de 5 à 1000 mg/jour.

12. Composition destinée à être utilisée selon l'une quelconque des revendications précédentes,
la composition étant utilisée comme agent thérapeutique combiné avec des corticostéroïdes, en particulier des corticostéroïdes systémiques ou topiques.

13. Médicament pour le traitement prophylactique et/ou thérapeutique des polypes nasaux (polypose naso-sinusienne),
le médicament contenant du cinéol, de préférence du 1,8-cinéol, comme principe actif, en particulier comme principe actif pharmaceutique.

14. Combinaison de principes actifs, en particulier sous la forme d'un kit (ensemble d'éléments), pour une utilisation dans le traitement prophylactique et/ou thérapeutique des polypes nasaux (polypose naso-sinusienne),
la combinaison de principes actifs comprenant des composants séparés physiquement, mais liés fonctionnellement et/ou destinés à la co-médication ou à la thérapie combinée, à savoir
(a) le cinéol, en particulier le 1,8-cinéol, de préférence sous la forme d'une composition pharmaceutique contenant le cinéol et destinée à être appliquée par voie topique ou systémique, d'une part et
(b) au moins un corticostéroïde, de préférence sous la forme d'une composition pharmaceutique contenant le corticostéroïde et destinée à être appliquée par voie topique ou
systémique.

15. Cinéol, en particulier 1,8-cinéol, destiné à être utilisé dans le traitement prophylactique et/ou thérapeutique de polypes nasaux (polypose naso-sinusienne), de polypes nasaux (polypose naso-sinusienne) associés et/ou induits et/ou favorisés par la voie de signalisation Wnt, en particulier la voie de transduction du signal Wnt/bêta-caténine, de préférence de formes récidivantes de polypes nasaux (polypose naso-sinusienne) ;
le cinéol étant utilisé comme régulateur, de préférence inhibiteur et/ou suppresseur, de la voie de signalisation Wnt, en particulier de la voie de transduction du signal Wnt/bêta-caténine, de préférence comme régulateur et/ou réducteur de la phosphorylation de la protéine kinase GSK-3 dans la voie de signalisation Wnt ou pour activer la protéine kinase GSK-3 et/ou comme désactivateur de la bêta-caténine dans la voie de signalisation Wnt ; et/ou
le cinéol étant utilisé comme régulateur, de préférence inhibiteur et/ou suppresseur, d'au moins un gène Wnt et/ou une protéine Wnt.
